# EUROPEAN PATENT APPLICATION

(11) **EP 2 921 835 A1**
(43) Date of publication of application: **23.09.2015**
(21) Application number: 15165976.0
(22) Date of filing: 01.10.2010
(51) Int. Cl.: G01L 5/00, G01L 1/00, A61B 17/12, A61B 17/00, A61B 19/00, A61M 25/01, G01L 5/10, G01L 1/04

(54) **INSERTION DEVICE, TRAINING DEVICE, AND RECORDING SYSTEM**

(30) Priority: 14.10.2009 JP 2009236869
(62) Divisional of application: 13198514.5
(71) Applicant: National University Corporation Nagoya Institute Of Technology, Nagoya-shi, Aichi 466-0061 (JP); National University Corporation Nagoya University, Nagoya-shi, Aichi 464-8601 (JP); NTN Corporation, Osaka-shi, Osaka 550-0003 (JP)
(72) Inventor: Fujimoto, Hideo, Nagoya-shi, Aichi 466-0061 (JP); Miyachi, Shigeru, Nagoya-shi, Aichi 464-8601 (JP); Nagano, Yoshitaka, Iwata-Shi, Shizuoka 438-8510 (JP); Ozaki, Takayoshi, Iwata-shi, Shizuoka 438-8510 (JP)
(74) Representative: Bockhorni & Kollegen

(57) **Abstract**

This insertion device is an insertion device for inserting a medical linear body (104) in a vessel in a body, and it includes a drive device (1) for moving the medical linear body (104) in a direction of a longitudinal axis, a measurement device (60) for measuring compressive force and pulling force in the direction of the longitudinal axis applied to the medical linear body (104), and a notification device (92 to 94) for notifying an operator of compressive force and pulling force measured with the measurement device (60).

## Description

### TECHNICAL FIELD

This invention relates to an insertion device, a training device, and a recording system, and particularly to an insertion device for inserting a medical linear body in a vessel in a body, a training device, and a recording system.

### BACKGROUND ART

A linear body having flexibility has been put into practical use as a linear medical appliance inserted in a vessel in a body. For example, a guide wire or a catheter inserted in a vessel in a body such as a blood vessel, a ureter, a bronchus, an alimentary canal, or a lymph vessel, or a wire having an embolus coil attached at a tip end for embolizing an aneurysm has been known. Such a linear body is inserted in a vessel in a body and guided to a destination through an operation from outside the body.

In many cases, the vessel in which the linear body is inserted is not necessarily linear but partially flexed or branched. In addition, a diameter of the vessel is not necessarily uniform, and the vessel itself may become thinner or a diameter of the vessel may be made smaller by an obstacle located in the vessel such as a thrombus in a blood vessel. A conventional linear body, however, has not been provided with means for sensing a condition in a direction of travel of the linear body, and it has been necessary to use operator's intuition in operating the linear body and the operator has had to be skilled in the operation for guiding the linear body from outside the body. A device provided with a pressure sensor at a tip end of a linear body is disclosed as a device sensing presence of an obstacle in a direction of travel of the linear body (see, for example, PTL 1 (Japanese Patent Laying-Open No. 10-263089)).

On the other hand, it is difficult to realize a device provided with a pressure sensor at the tip end of a linear body, in particular when the linear body is extremely thin. For example, a guide wire to be inserted in a cerebral blood vessel has a diameter around 0.35 mm, and it is difficult to provide a small pressure sensor at the tip end of such an extremely thin linear body. In addition, it is more difficult to insert a wire into the linear body in order to extract a signal from the pressure sensor to the outside.

Moreover, if the vessel in which the linear body is inserted is flexed or if a diameter of the vessel is small, insertion resistance of the linear body is affected by friction with the vessel. Accordingly, an output from the pressure sensor provided at the tip end of the linear body may not necessarily be in agreement with kinesthetic sense of the operator at the time of insertion. Therefore, even when the device provided with the pressure sensor at the tip end of the linear body is used, the operator operates the linear body based on kinesthetic sense information of the insertion resistance of the linear body externally held with fingers of the operator, that is, relying on intuition of the operator. Further, as it is only the operator that can feel the kinesthetic sense, it is difficult to quantify manipulation of a skilled operator so as to transfer the skill to a less experienced operator.

In addition, it is not cost effective to prepare linear bodies of various shapes and materials for adaptation to different applications and to provide pressure sensors in respective linear bodies, and manufacturing cost is increased.

As a technique for solving such problems, for example, PTL 2 (Japanese Patent Laying-Open No. 2007-292711) discloses a construction as follows. Namely, a measurement device for measuring compressive force in a direction of a longitudinal axis applied to a linear body having flexibility includes a main body in which a through hole through which the linear body passes is formed, the linear body bending in a prescribed direction within the through hole when the compressive force is applied to the linear body, a sensor for detecting the degree of bending, and a conversion circuit for converting the detected degree of bending into the compressive force applied to the linear body.

The constructions described in PTLs 1 and 2 are for measuring compressive force applied to the linear body. Meanwhile, as a technique for measuring pulling force applied to a linear body, for example, PTL 3 (Japanese Patent Laying-Open No. 2002-90239) discloses a construction as follows. Namely, a linear body tension detection device for detecting pulling force applied to a linear body includes an attachment portion freely attachable to/removable from a support portion intermediate in the linear body with movement of the linear body being allowed, which is provided at each of opposing end portions of the support portion, a flexure portion provided in a central portion of the attachment portion and projecting in a direction orthogonal to the linear body, for flexing the linear body, and a detector for detecting force applied by the linear body when the linear body flexed by this flexure portion returns to a straight shape, so that pulling force is detected based on force detected by the detector.

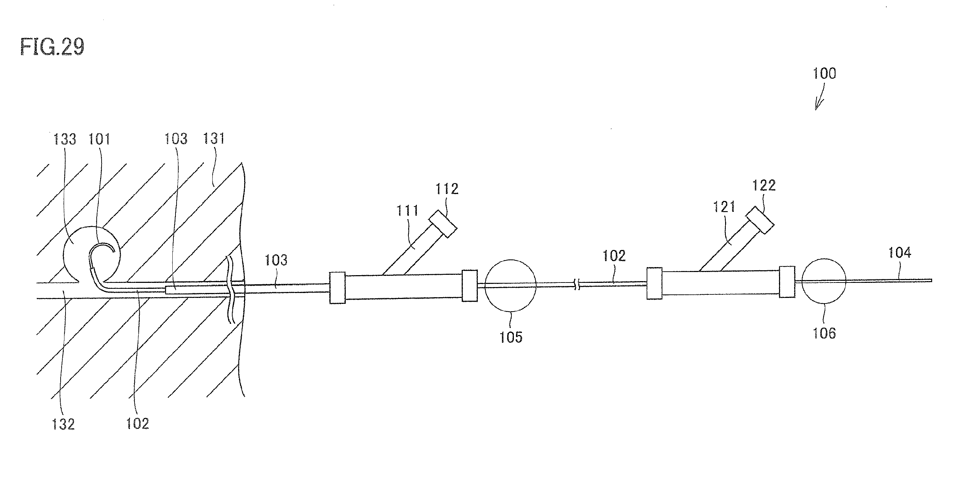

Recently, a minimally invasive surgical operation such as treatment using a catheter has been performed. Treatment using a catheter includes, for example, coil embolization treatment. The coil embolization treatment refers to treatment for preventing rupture of a cerebral aneurysm representing a cause of subarachnoid hemorrhage by leaving a coil in the cerebral aneurysm for embolization. Fig. 29 is a schematic diagram showing a medical appliance used for the coil embolization treatment.

In a medical appliance 100 shown in Fig. 29, a coil 101 made of platinum for embolizing a cerebral aneurysm 133 with a coil is connected at a tip end of a delivery wire 104. A catheter into which delivery wire 104 is inserted is a double-lumen catheter having a guiding catheter 103 as an outer lumen and having a microcatheter 102 as an inner lumen. Microcatheter 102 is hollow and delivery wire 104 is inserted in the hollow portion of microcatheter 102. Delivery wire 104 is inserted in a Y-connector 121 and microcatheter 102 is inserted in a Y-connector 111.

A doctor operates delivery wire 104 at a holding portion 106 around an inlet port of Y-connector 121. Another doctor operates catheter 102 at a holding portion 105 around an inlet port of Y-connector 111. Namely, two doctors operate delivery wire 104 and microcatheter 102 around the inlet ports of Y-connectors 111, 121, respectively.

Y-connector 111, 121 has three connection ports. One is a catheter connection port, another one is a port in which such a linear body as a catheter or a delivery wire is inserted, and another one is an input port 112, 122 for physiological saline or a medicine.

Guiding catheter 103 is inserted in a blood vessel 132 of a human body 131 and its tip end reaches a portion in the vicinity of cerebral aneurysm 133. Microcatheter 102 is inserted in the inside of guiding catheter 103 and it is advanced from a tip end of guiding catheter 103 into cerebral aneurysm 133. Coil 101 is pushed out of microcatheter 102 that has reached the inside of cerebral aneurysm 133 and cerebral aneurysm 133 is filled with thin and soft coil 101. Rupture of cerebral aneurysm 133 is thus prevented.

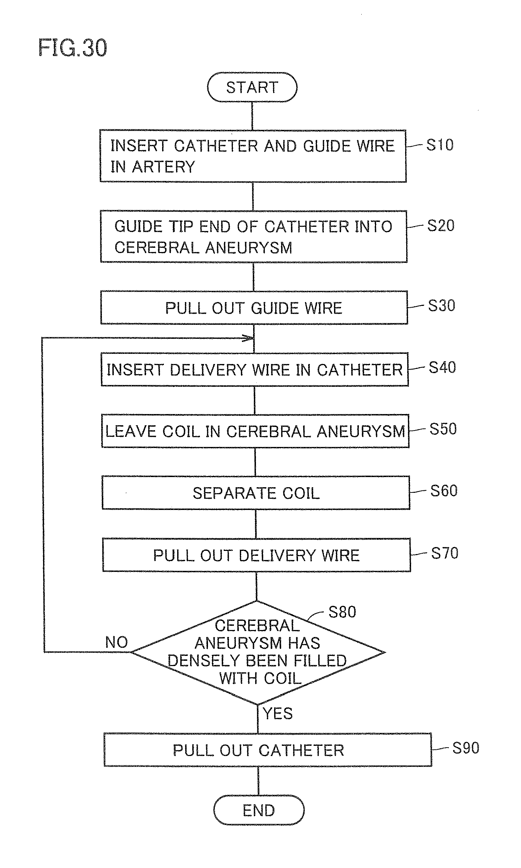

Fig. 30 is a flowchart showing a procedure of the coil embolization treatment. The coil embolization treatment is generally provided in a procedure shown in Fig. 30. Initially, in a step (S10), two catheters (guiding catheter 103 and microcatheter 102) in a double-lumen structure and a guide wire used for guiding the catheters to a destination are inserted in an artery in a femoral region. Microcatheter 102 is inserted in guiding catheter 103 and the guide wire is inserted in microcatheter 102. Then, in a step (S20), a tip end of microcatheter 102 is guided by the guide wire and placed in cerebral aneurysm 133.

Then, in a step (S30), the guide wire is pulled out of microcatheter 102. In succession, in a step (S40), delivery wire 104 provided with coil 101 made of platinum at the tip end is inserted in microcatheter 102 in place of the guide wire.

Then, in a step (S50), coil 101 is left in cerebral aneurysm 133. Thereafter, in a step (S60), an electrode is connected to delivery wire 104 and an electrode is also connected to a needle inserted in advance in human body 131, and thereafter a current is fed between delivery wire 104 and human body 131 through these electrodes. Since coil 101 and delivery wire 104 are connected to each other through a material that is decomposed by an electric current, coil 101 and delivery wire 104 are separated from each other by feeding power, and consequently coil 101 is left in cerebral aneurysm 133.

Then, in a step (S70), delivery wire 104 is pulled out of microcatheter 102. Thereafter, in a step (S80), whether cerebral aneurysm 133 is densely filled with coil 101 or not is determined. When it is determined that cerebral aneurysm 133 is not densely filled with coil 101, the process returns to the step (S40) and delivery wire 104 provided with another coil 101 is inserted in microcatheter 102. The steps (S40) to (S70) are repeated until cerebral aneurysm 133 is densely filled with coil 101.

When it is determined that cerebral aneurysm 133 is densely filled with coil 101, guiding catheter 103 and microcatheter 102 are then pulled out of human body 131 in a step (S90). The coil embolization treatment of cerebral aneurysm 133 is thus completed.

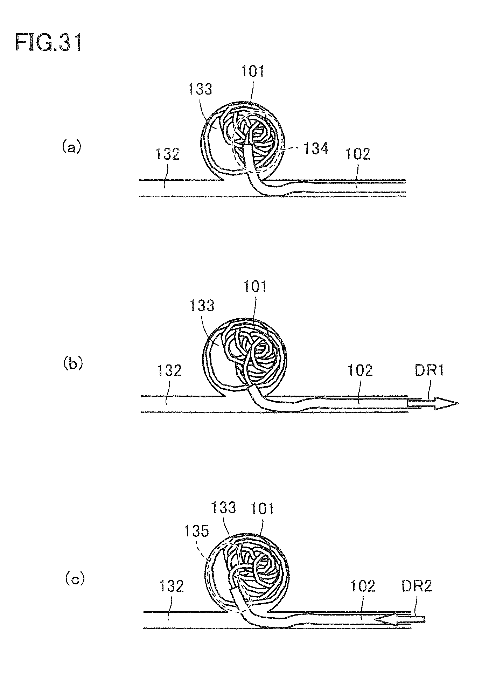

Fig. 31 is a schematic diagram showing an operation of a catheter for leaving a coil in a cerebral aneurysm. As shown in Fig. 31(a), when cerebral aneurysm 133 is embolized by leaving coil 101 connected to the tip end of delivery wire 104 in cerebral aneurysm 133, coil 101 may be located in the inside of cerebral aneurysm 133 in an unbalanced manner and density of coil 101 in the vicinity of the tip end portion of microcatheter 102 may be high. If the tip end portion of microcatheter 102 is located in a high-coil-density region 134, resistance in inserting coil 101 in cerebral aneurysm 133 becomes high.

As delivery wire 104 is inserted further in blood vessel 132 while insertion resistance is high, rupture of cerebral aneurysm 133 of which blood vessel wall is thin and brittle may be caused. Therefore, when high resistance in inserting coil 101 is sensed, a doctor operating delivery wire 104 temporarily stops insertion of delivery wire 104.

In addition, a doctor who operates microcatheter 102 moves back microcatheter 102. Namely, as shown in Fig. 31(b), the doctor who operates microcatheter 102 moves microcatheter 102 in a direction DR1 of pull-out from blood vessel 132. Thereafter, the doctor who operates microcatheter 102 again advances microcatheter 102. Namely, the doctor who operates microcatheter 102 moves microcatheter 102 in a direction DR2 of insertion in blood vessel 132, as shown in Fig. 31(c).

As a result of an operation to once move back microcatheter 102 and again advance the same, a position of the tip end portion of microcatheter 102 is changed and the tip end portion of microcatheter 102 moves in a low-coil-density region 135 in cerebral aneurysm 133. As density of coil 101 in the vicinity of the tip end portion of microcatheter 102 is lower, resistance in inserting coil 101 in cerebral aneurysm 133 becomes lower. In this state, the doctor who operates delivery wire 104 resumes insertion of delivery wire 104, that is, insertion of coil 101 in cerebral aneurysm 133.

Thus, since delicate control is required in operating catheter 102, 103 or delivery wire 104 in catheter treatment, an operator should be skilled. Then, in order to improve operability of catheter 102, 103 or delivery wire 104 in catheter treatment, several master-slave-type drive devices have been proposed (see, for example, PTL 4 (Japanese Patent Laying-Open No. 2000-42116) and PTL 5 (Japanese Patent Laying-Open No. 2001-157662)).

### CITATION LIST

### PATENT LITERATURE

PTL 1: Japanese Patent Laying-Open No. 10-263089
PTL 2: Japanese Patent Laying-Open No. 2007-292711
PTL 3: Japanese Patent Laying-Open No. 2002-90239
PTL 4: Japanese Patent Laying-Open No. 2000-42116
PTL 5: Japanese Patent Laying-Open No. 2001-157662

### SUMMARY OF INVENTION

### TECHNICAL PROBLEM

The constructions described in PTLs 1 to 3, however, can detect only one of compressive force and pulling force on the linear body.

Measurement of compressive force applied to the linear body is a function necessary for avoiding damage to a human body as a result of application of excessive compressive force, that is, insertion force, to the linear body introduced in a body.

In addition, in embolization of a cerebral aneurysm with a coil, in feeding a treatment device into a body through a linear body, alignment of the treatment device or change of the treatment device to another device of a different size for better adaptation may be made. In this case, an operation for pulling back the linear body is performed. If the linear body cannot smoothly be pulled back, however, it is expected that the treatment device or the linear body inserted in the body is caught in the body. If the linear body is forcibly pulled back in such a case, excessive load will be applied to the human body, the treatment device, and the linear body, which is not preferred.

In addition, in a master-slave-type drive device, a required operation is different from a case where such a linear body as delivery wire 104 or catheter 102, 103 is manually operated. Therefore, an operator should newly be trained. Moreover, in using a master-slave device, it is difficult to feel small change in patient's heartbeat, blood vessel 132, or the like. Therefore, the operator preferably manually operates the linear body while holding the same.

As described above, in conventional catheter treatment, two doctors holding microcatheter 102 and deliver wire 104 respectively provide treatment in cooperation with each other. If any one is different in manipulation or in manipulation level, however, it may take time to establish sufficient cooperation. In addition, a doctor may feel stressful in order to establish cooperation. In the drive device described in PTL 4, an operation of a catheter and a wire used as the linear body is the same as in conventional surgical operations described above, and hence the linear bodies should be operated by two respective doctors. Means for solving this problem, however, has not been proposed.

Further, in a master-slave system, another problem concerning a scaling function for improving operability has arisen. A scaling function refers to a function to change a ratio of an amount of travel of a linear body on a slave side with respect to an amount of operation on a master side to thereby achieve a scale that can easily be handled by a person in performing a delicate operation. When it is assumed that a scale value is N-fold, an amount of travel on the slave side with respect to an operation on the master side is 1/N-fold, which is a reciprocal of the scale value, and here, a length of an operation portion of the linear body on the master side is N times as great as the amount of travel of the linear body on the slave side. Therefore, an operation portion having a length in proportion to a maximum scale value should be prepared. A catheter and a wire, however, each have a length from 1 m to 2 m, and hence such an operation portion is unrealistic. Further, since operated linear bodies are concentrically layered, the inner linear body should be operated at a portion where the outer linear body is not present. Then, a distance between two doctors who perform an operation becomes greater in proportion to the scale value, which leads to further difficulty in cooperation between the doctors.

Furthermore, in a case of a medical appliance, cleanliness should be maintained and a portion in contact with a human body is desirably disposable. For disposability, low cost is required, however, a master-slave system great in the number of parts is expensive.

The present invention was made in view of the problems above, and a main object thereof is to provide an insertion device with which application of excessive compressive force (insertion force) and pulling force (pull-out force) to a medical linear body is prevented with a simplified construction and which can be operated by one doctor, a training device, and a recording system.

### SOLUTION TO PROBLEM

In order to solve the problems above, an insertion device according to one aspect of this invention is an insertion device for inserting a medical linear body in a vessel in a body, and it includes a drive device for moving the medical linear body in a direction of a longitudinal axis, a measurement device for measuring compressive force and pulling force in the direction of the longitudinal axis applied to the medical linear body, and a notification device for notifying an operator of compressive force and pulling force measured with the measurement device.

Preferably, the insertion device further includes a control device for controlling, when compressive force measured with the measurement device exceeds a first threshold value or when pulling force measured with the measurement device exceeds a second threshold value, the drive device so as to reduce a moving speed of the medical linear body in accordance with an amount of excess of the compressive force and the pulling force over the respective first and second threshold values or stop movement of the medical linear body.

Further preferably, the insertion device further includes a Y-connector for inserting the medical linear body in a catheter. The drive device is arranged on a side of a port for insertion of the medical linear body in the Y-connector and is attachable to and removable from the Y-connector.

Further preferably, the medical linear body is a delivery wire provided with an embolus coil at a tip end.

Further preferably, the insertion device further includes a foot switch for starting and stopping movement of the medical linear body by the drive device in response to an operation by the operator.

Further preferably, the notification device notifies the operator of compressive force and pulling force applied to the medical linear body through light or sound and changes luminance of light, a wavelength of light, a frequency of light, the number of light beams intermittently emitted per a prescribed time period, sound volume, a wavelength of sound, a frequency of sound, or the number of sounds intermittently emitted per a prescribed time period in accordance with magnitude of compressive force and pulling force measured with the measurement device.

Further preferably, the measurement device includes a main body in which a through hole for inserting the medical linear body is formed. The through hole is formed to allow bending of the medical linear body in an arc shape in the through hole and variation in degree of bending of the medical linear body in accordance with the compressive force and the pulling force. The measurement device further includes a sensor for detecting a degree of bending of the medical linear body and a conversion circuit for converting the degree of bending detected by the sensor into a signal indicating compressive force and pulling force applied to the medical linear body. The conversion circuit converts the detected degree of bending into a signal indicating compressive force applied to the medical linear body when the degree of bending of the medical linear body increases as compared with the degree of bending of the medical linear body while compressive force and pulling force are not applied to the medical linear body, and converts the detected degree of bending into a signal indicating pulling force applied to the medical linear body when the degree of bending of the medical linear body decreases as compared with the degree of bending of the medical linear body while compressive force and pulling force are not applied to the medical linear body.

Further preferably, the insertion device further includes a Y-connector for inserting the medical linear body in a catheter, and the measurement device is integrated with the Y-connector.

Further preferably, the sensor is an optical line sensor including a light receiver receiving light emitted by a light source, for detecting the degree of bending of the medical linear body by detecting a position at which a quantity of light received by the light receiver decreases as the medical linear body cuts off the light emitted by the light source.

Further preferably, the insertion device further includes a Y-connector for inserting the medical linear body in a catheter, and the Y-connector is attachable to and removable from the line sensor.

Further preferably, the line sensor and the drive device are integrated with each other with a base interposed therebetween.

Further preferably, the drive device includes a rotational force generator, a drive roller rotatably driven by rotational force generated by the rotational force generator, a driven roller rotated as the drive roller rotates, a case containing the rotational force generator, the drive roller, and the driven roller, and an elastic body provided between the case and the driven roller. The medical linear body is sandwiched between a rotation surface of the drive roller and a rotation surface of the driven roller, and the driven roller is supported by the case with the elastic body being interposed, and pressed against the drive roller by elastic force from the elastic body. Pressing of the driven roller against the drive roller can manually be removed.

Further preferably, the drive device includes a rotational force generator for generating rotational force for moving the medical linear body in the direction of the longitudinal axis based on a supplied drive current and a current detector for detecting the drive current. The measurement device measures compressive force and pulling force applied to the medical linear body based on the drive current detected by the current detector.

Further preferably, the insertion device further includes a linear body speed instruction portion for controlling a speed of the medical linear body moved by the drive device in response to an operation by the operator.

In order to solve the problems above, a training device according to one aspect of this invention includes the insertion device above.

In order to solve the problems above, a recording system according to one aspect of this invention includes an insertion device for inserting a medical linear body in a vessel in a body and a recording device, and the insertion device includes a drive device for moving the medical linear body in a direction of a longitudinal axis, a measurement device for measuring compressive force and pulling force in the direction of the longitudinal axis applied to the medical linear body, and a notification device for notifying an operator of compressive force and pulling force measured with the measurement device. The recording device records an image obtained by photographing the medical linear body as well as compressive force and pulling force measured with the measurement device, temporally in correspondence with each other.

Preferably, the recording device records the image, the compressive force and pulling force, and a speed of the medical linear body moved by the drive device, temporally in correspondence with one another.

### ADVANTAGEOUS EFFECTS OF INVENTION

According to the present invention, application of excessive compressive force (insertion force) and pulling force (pull-out force) to a medical linear body can be prevented with a simplified construction and one doctor can perform an operation.

### BRIEF DESCRIPTION OF DRAWINGS

Fig. 1 is a schematic diagram showing a construction of an insertion device for inserting a delivery wire in a blood vessel according to Embodiment 1 of the present invention.
Fig. 2 is a schematic cross-sectional view of a drive device for a medical linear body.
Fig. 3 is a schematic partial cross-sectional view of the drive device along the line III-III shown in Fig. 2.
Fig. 4 is a schematic cross-sectional view of the drive device along the line IV-IV shown in Fig. 2.
Fig. 5 is a side view of the drive device shown in Figs. 2 to 4.
Fig. 6 is an external view showing a construction of a main body of a measurement device according to Embodiment 1 of the present invention.
Fig. 7 is a cross-sectional view showing a cross-section along the line VII-VII in Fig. 6.
Fig. 8 is a cross-sectional view showing a cross-section along the line VIII-VIII in Fig. 6.
Fig. 9 is a schematic diagram showing an overall configuration of the measurement device.
Fig. 10 is a diagram showing such a state that compressive force is applied to a delivery wire 104 and delivery wire 104 is bent in a main body 52 in the cross-sectional view in Fig. 7.
Fig. 11 is a diagram showing such a state that pulling force is applied to delivery wire 104 and delivery wire 104 is bent in main body 52 in the cross-sectional view in Fig. 7.
Fig. 12 is a diagram showing correlation between force applied to the linear body and a position of the linear body detected by a line sensor.
Fig. 13 is a diagram showing a procedure of a measurement method according to Embodiment 1 of the present invention.
Fig. 14 is a diagram showing a construction of a Y-connector according to Embodiment 1 of the present invention.
Fig. 15 is a diagram showing acoustic control carried out by a sensor output control device in the insertion device according to Embodiment 1 of the present invention.
Fig. 16 is a diagram showing acoustic control carried out by the sensor output control device in the insertion device according to Embodiment 1 of the present invention.
Fig. 17 is a diagram showing acoustic control carried out by the sensor output control device in the insertion device according to Embodiment 1 of the present invention.
Fig. 18 is a diagram showing a construction of a coil for embolization of a cerebral aneurysm with a coil.
Fig. 19 is a cross-sectional view showing a construction of a measurement device according to Embodiment 2 of the present invention.
Fig. 20 is a cross-sectional view showing a cross-section along the line XX-XX in Fig. 19.
Fig. 21 is a diagram showing such a state that a sheath 85 is inserted into main body 52 in the cross-sectional view in Fig. 19.
Fig. 22 is a diagram showing a construction of an insertion device according to Embodiment 3 of the present invention.
Fig. 23 is a diagram showing the construction of the insertion device according to Embodiment 3 of the present invention.
Fig. 24 is a diagram showing a configuration of a drive device in an insertion device according to Embodiment 4 of the present invention.
Fig. 25 is a diagram showing a configuration of a linear body operation recording system according to Embodiment 5 of the present invention.
Fig. 26 is a diagram showing a separated structure of a Y-connector, a measurement device, and a drive device in an insertion device according to Embodiment 6 of the present invention.
Fig. 27 is a diagram showing a separated structure of a Y-connector, a measurement device, and a drive device in an insertion device according to Embodiment 7 of the present invention.
Fig. 28 is a diagram showing a construction of a training device according to Embodiment 8 of the present invention.
Fig. 29 is a schematic diagram showing a medical appliance used for coil embolization treatment.
Fig. 30 is a flowchart showing a procedure of the coil embolization treatment.
Fig. 31 is a schematic diagram showing an operation of a catheter for leaving a coil in a cerebral aneurysm.

### DESCRIPTION OF EMBODIMENTS

An embodiment of the present invention will be described hereinafter with reference to the drawings. In the drawings, the same or corresponding elements have the same reference characters allotted and description thereof will not be repeated.

It is noted that, in the embodiment which will be described below, each component is not necessarily essential in the present invention, unless otherwise specified. In addition, when the number, an amount or the like is mentioned in the embodiment below, the number or the like above is by way of example and the scope of the present invention is not necessarily limited to the number, the amount or the like, unless otherwise specified.

### <Embodiment 1>

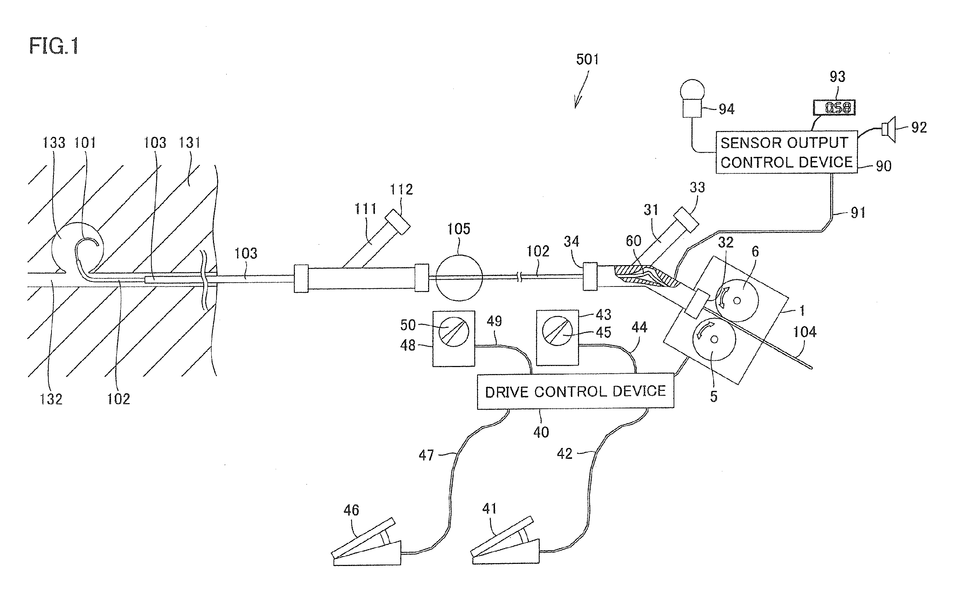

Fig. 1 is a schematic diagram showing a construction of an insertion device for inserting a delivery wire into a blood vessel according to Embodiment 1 of the present invention. An insertion device for inserting delivery wire 104 inserted into catheter 102, 103 in blood vessel 132 in a body, that is used in coil embolization treatment, will be described with reference to Fig. 1.

As shown in Fig. 1, delivery wire 104 representing a medical linear body is inserted in a Y-connector 31. Coil 101 made of platinum for embolizing cerebral aneurysm 133 with a coil is connected to a head of delivery wire 104. Delivery wire 104 is inserted in Y-connector 31 through a first input port 32, it passes through Y-connector 31, and it is inserted in microcatheter 102 connected to an output port 34. It is noted that a construction of the insertion device from output port 34 of Y-connector 31 to the inside of human body 131 is the same as that of conventional medical appliance 100 shown in Fig. 29 and hence description thereof will not be repeated.

An insertion device 501 includes a drive device 1 for moving delivery wire 104 in a direction of a longitudinal axis. Drive device 1 is arranged on a side of a port of insertion of delivery wire 104 in Y-connector 31 and it is attachable to and removable from Y-connector 31. Drive device 1 includes a drive roller 5 and a driven roller 6. Delivery wire 104 is sandwiched between a rotation surface of drive roller 5 and a rotation surface of driven roller 6 and it moves in the direction of the longitudinal axis as drive roller 5 rotates. Movement of delivery wire 104 in the direction of the longitudinal axis by drive device 1 is controlled by a drive control device 40. To drive control device 40, an insertion foot switch 41 is electrically connected through a line 42 and a pull-out foot switch 46 is electrically connected through a line 47.

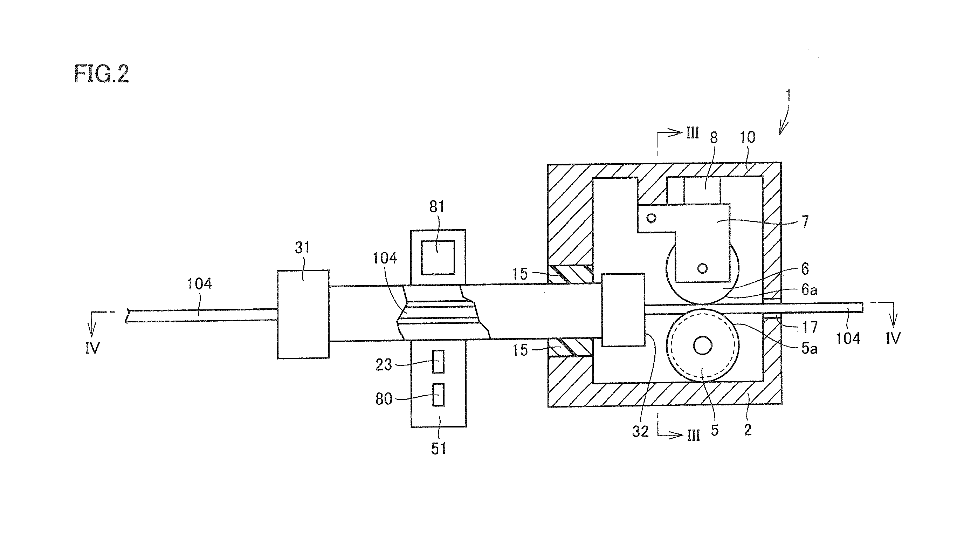

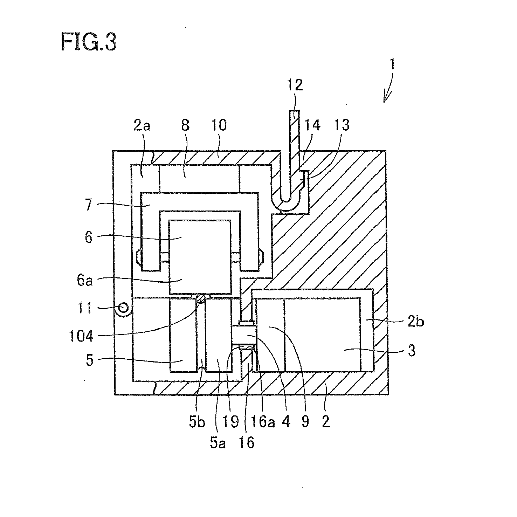

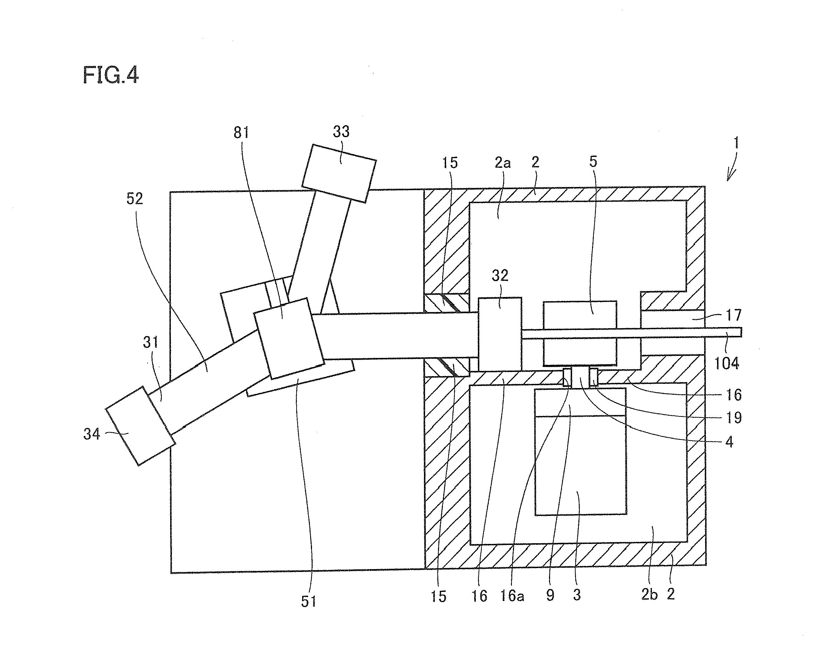

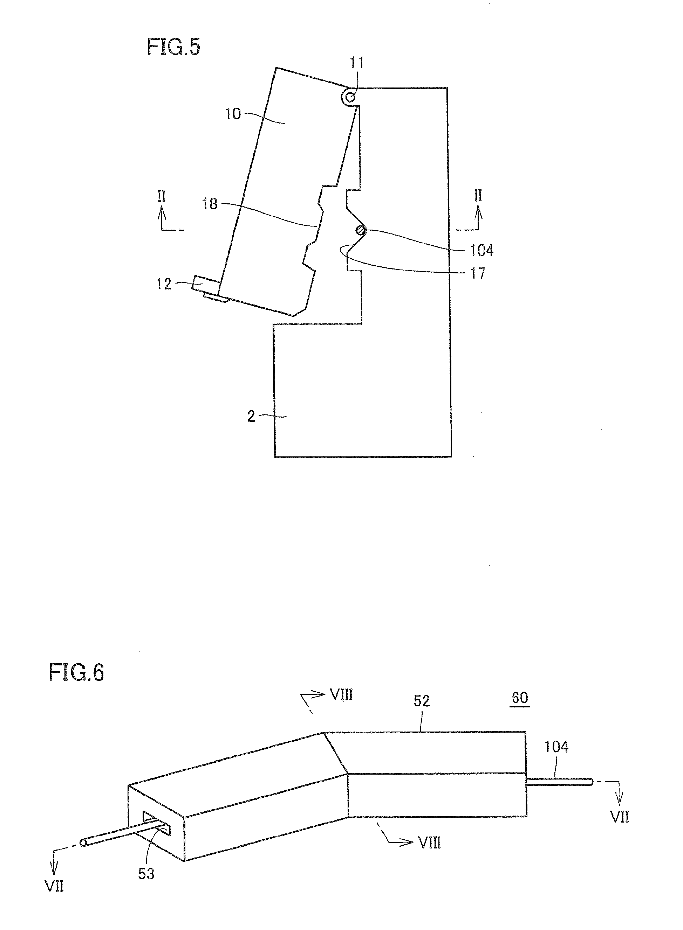

Fig. 2 is a schematic cross-sectional view of the drive device for a medical linear body. Fig. 3 is a schematic partial cross-sectional view of the drive device along the line III-III shown in Fig. 2. Fig. 4 is a schematic cross-sectional view of the drive device along the line IV-IV shown in Fig. 2. Fig. 5 is a side view of the drive device shown in Figs. 2 to 4. A construction of drive device 1 for a medical linear body (delivery wire 104) will be described with reference to Figs. 2 to 5. It is noted that Fig. 2 is a cross-sectional view of the drive device along the line II-II shown in Fig. 5.

As shown in Figs. 2 to 5, drive device 1 includes a housing 2. Housing 2 has a lid member 10 provided to be opened and closed as it pivotably moves with a hinge 11 serving as a pivot. A partition wall 16 is provided in an internal space of drive device 1 formed as surrounded by housing 2 and lid member 10. Partition wall 16 partitions the internal space of drive device 1 into a large chamber 2a which is a first space and a small chamber 2b which is a second space. In small chamber 2b, a motor 3 serving as a rotational force generator and a speed reduction device 9 for lowering a rotation speed of rotational force generated by motor 3 and outputting the resultant rotational force are arranged. Motor 3 is an electric motor converting electric energy into mechanical energy.

A hole 16a penetrating partition wall 16 in a direction of thickness is formed in partition wall 16. A rotation shaft 4 serving as a rotation portion is arranged to pass through hole 16a. Rotation shaft 4 transmits rotational force generated by motor 3, of which rotation speed has been reduced by speed reduction device 9, to drive roller 5.

A case forming an outer shell of small chamber 2b is formed from a part of housing 2 and partition wall 16. The part of housing 2 and partition wall 16 form a wall portion of the case containing motor 3 and speed reduction device 9. As shown in Figs. 3 and 4, the case is formed in a parallelepiped box shape, partition wall 16 forms one surface of wall surfaces of the case, and housing 2 forms three surfaces of a bottom surface, a ceiling surface, and a wall surface of the case. Hole 16a through which rotation shaft 4 passes is formed in partition wall 16 forming a part of the case.

A sealing portion 19 is provided on an inner circumferential surface of hole 16a formed in partition wall 16. Sealing portion 19 is formed to be in contact with the inner circumferential surface of hole 16a and in contact with an outer circumferential surface of rotation shaft 4. Sealing portion 19 closes a gap between partition wall 16 and rotation shaft 4 and cuts off small chamber 2b which is the inside of the case above from the outside. Though small chamber 2b in which motor 3 and speed reduction device 9 are arranged communicates with large chamber 2a only through hole 16a, large chamber 2a and small chamber 2b are provided as separate spaces, because sealing portion 19 closes hole 16a. As sealing portion 19 closes hole 16a, small chamber 2b is provided as a hermetically sealed space. Sealing portion 19 suppresses leakage of a liquid from large chamber 2a to small chamber 2b through hole 16a.

In large chamber 2a, drive roller 5 carrying out rotational motion with rotational force generated by motor 3 and transmitted through rotation shaft 4 is provided. Drive roller 5 is a feed roller attached to motor 3 with rotation shaft 4 and speed reduction device 9 being interposed, and it is formed substantially in a cylindrical shape. Speed reduction device 9 is interposed between motor 3 and drive roller 5. Rotation shaft 4 transmits rotational force from speed reduction device 9 to drive roller 5. A feed groove 5b is formed in a rotation surface 5a which is a side surface of drive roller 5 in a cylindrical shape. Feed groove 5b is in a V shape or has an appropriate curvature.

In addition, in large chamber 2a, driven roller 6 is arranged to be opposed to rotation surface 5a of drive roller 5. Driven roller 6 serving as a pressure roller for applying a pressure to delivery wire 104 is formed substantially in a cylindrical shape. Delivery wire 104 is sandwiched between a rotation surface 6a of driven roller 6 which is the side surface of the cylindrical shape and rotation surface 5a of drive roller 5. Rotation surface 5a of drive roller 5 and rotation surface 6a of driven roller 6 are located to be opposed to each other, with delivery wire 104 lying therebetween. Delivery wire 104 is arranged between rotation surfaces 5a and 6a so as to extend along feed groove 5b formed in rotation surface 5a of drive roller 5.

As motor 3 is started and drive roller 5 carries out rotational motion, driven roller 6 carries out rotational motion following rotation of drive roller 5. As drive roller 5 and driven roller 6 rotate in directions reverse to each other, delivery wire 104 moves in the direction of the longitudinal axis of delivery wire 104. Delivery wire 104 is driven by drive roller 5. Motor 3 generating rotational force, speed reduction device 9 and rotation shaft 4 transmitting rotational force, and drive roller 5 and driven roller 6 carrying out rotational motion are included in an actuator serving as a feeding device for moving delivery wire 104 in the direction of the longitudinal axis thereof. The actuator moves delivery wire 104 in such a manner that it holds delivery wire 104 and sends delivery wire 104 in the direction of the longitudinal axis thereof. The actuator is arranged in the internal space of housing 2 and held by housing 2.

When driven roller 6 is pressed against drive roller 5 to thereby hold delivery wire 104, rotation surface 5a of drive roller 5 and rotation surface 6a of driven roller 6 are desirably formed such that damage to delivery wire 104 can be suppressed and delivery wire 104 can smoothly be moved. For example, stainless steel can be employed as a material for drive roller 5 and driven roller 6, and a urethane resin or the like can be used as a material for coating of rotation surface 5a, 6a.

As rotation surface 5a of drive roller 5 and rotation surface 6a of driven roller 6 are formed of an elastic material such as a urethane resin, a surface of delivery wire 104 can be in surface contact with rotation surface 5a, 6a so that friction force generated between rotation surface 5a, 6a and delivery wire 104 can be increased. With this friction force, even though compressive force applied to delivery wire 104 in the direction of the longitudinal axis increases in moving delivery wire 104, slipping of delivery wire 104 with respect to rotation surface 5a, 6a can be suppressed. In addition, since feed groove 5b is formed in rotation surface 5a of drive roller 5, an area of contact between delivery wire 104 and rotation surface 5a increases. Formation of this feed groove 5b can also increase friction force generated between rotation surface 5a, 6a and delivery wire 104.

Though Fig. 3 illustrates an example where feed groove 5b is formed in rotation surface 5a of drive roller 5, the construction is not limited as such. Rotation surface 5a of drive roller 5 may be formed as a smooth curved surface without irregularities, with no groove being formed, and a feed groove in which the linear body is to be arranged may be formed in rotation surface 6a of driven roller 6. Alternatively, a feed groove may be formed in each of rotation surfaces 5a and 6a. Namely, so long as the construction is such that a groove portion is formed in rotation surface 5a, 6a of at least any one of drive roller 5 and driven roller 6, delivery wire 104 is arranged in the groove portion, the surface of delivery wire 104 comes in surface contact with an inner surface of the groove portion and an area of contact between delivery wire 104 and rotation surface 5a, 6a can be increased to thereby increase friction force so that an effect to suppress slipping of delivery wire 104 can be obtained.

Driven roller 6 is supported by lid member 10 in large chamber 2a in the internal space of drive device 1, with a support member 7 for rotatably supporting driven roller 6 and an elastic body 8 being interposed. Driven roller 6 is supported as suspended from lid member 10. Elastic body 8 is attached to lid member 10. Driven roller 6 is supported by lid member 10, for example, with such an elastic body 8 as rubber being interposed therebetween.

Lid member 10 has a lever 12 for operating opening and closing of lid member 10. Lever 12 is formed to be elastically deformable. Lever 12 shown in Fig. 3 has a substantial U shape. Lever 12 can elastically be deformed so as to decrease or increase a width of the U shape. A protrusion 13 is formed on lever 12. As this protrusion 13 is engaged with an engagement portion 14 of housing 2, lever 12 is fixed to housing 2.

An elastic portion 15 is provided in housing 2. In addition, elastic portion 15 is provided in lid member 10. As shown in Fig. 2, Y-connector 31 is fixed as it is sandwiched between elastic portion 15 on a housing 2 side and elastic portion 15 on a lid member 10 side. Housing 2 holds Y-connector 31 serving as the medical appliance. In Y-connector 31, a through hole passing through the inside thereof from first input port 32 to output port 34 is formed. Delivery wire 104 is inserted into the through hole in Y-connector 31. In addition, in Y-connector 31, another through hole passing through the inside thereof from second input port 33 to output port 34 is formed.

Y-connector 31 is a fixed member that is fixed by a fixing portion of drive device 1. The fixing portion has a hole shape formed at an abutment portion between a sidewall of housing 2 and a sidewall of lid member 10, and elastic portion 15 such as rubber provided on the inner circumferential side of the hole shape. Drive device 1 can fix Y-connector 31, which is a fixed member, in the fixing portion above. Y-connector 31 is attached to the fixing portion as it is sandwiched between elastic portions 15 attached to housing 2 and lid member 10 respectively. Elastic portion 15 is held by housing 2.

A guide groove 17 is formed at an abutment portion between another sidewall of housing 2 and another sidewall of lid member 10, opposed to one sidewall of housing 2 and one sidewall of lid member 10 where the fixing portion capable of fixing Y-connector 31 is provided. Guide groove 17 is formed in another sidewall of housing 2 opposed to the sidewall of housing 2 where elastic portion 15 is provided, at the portion of abutment to lid member 10. A projection portion 18 in a shape fitted to guide groove 17 is formed in another sidewall above of lid member 10. Guide groove 17 is formed by cutting a part of another sidewall above of housing 2.

While lid member 10 is closed, projection portion 18 is fitted into guide groove 17. Here, a space surrounded by projection portion 18 and guide groove 17 is slightly greater in diameter than delivery wire 104, so that delivery wire 104 can be inserted into the space above. Namely, when lid member 10 is closed and delivery wire 104 and Y-connector 31 are integrally attached to the fixing portion of drive device 1, delivery wire 104 is positioned by guide groove 17. Drive device 1 is provided with guide groove 17 for passage of delivery wire 104, that serves as a guide portion for positioning delivery wire 104.

A deepest portion of guide groove 17, which is a position where delivery wire 104 is to be placed, is formed around a position where an extension of the through hole formed in Y-connector 31 crosses another sidewall above of housing 2 and lid member 10 (typically, such that a direction in which the deepest portion of guide groove 17 passing through another sidewall above of housing 2 extends is coincident with a direction in which the through hole in Y-connector 31 extends).

By closing lid member 10 of drive device 1 where the guide portion is provided, delivery wire 104 is positioned and delivery wire 104 is set in place. Namely, when lid member 10 is closed, delivery wire 104 is correctly set between drive roller 5 and driven roller 6. Therefore, such defects as delivery wire 104 being sandwiched in the portion of abutment between lid member 10 and housing 2 resulting in interfered movement of delivery wire 104 or damage to delivery wire 104 can be suppressed.

Guide groove 17 may be formed in a V shape as shown in Fig. 5, or may be formed in a curved surface shape having an appropriate curvature, such as a U shape or an arc shape.

An operation for opening and closing lid member 10 of drive device 1 will be described below. By elastically deforming lever 12 to decrease the width of the U shape from the state that lid member 10 is closed shown in Figs. 2 and 3, protrusion 13 and engagement portion 14 are disengaged from each other. As protrusion 13 is disengaged from engagement portion 14, lid member 10 is now pivotable around hinge 11 serving as a pivot axis. In the cross-sectional view shown in Fig. 3, lid member 10 is opened by moving lid member 10 counterclockwise around hinge 11 serving as a pivot center. The shape is such that, as the operator holds and operates lever 12, he/she can manually open lid member 10. By elastically deforming lever 12 to thereby disengage protrusion 13 and engagement portion 14 from each other, lid member 10 can be opened.

As lid member 10 is opened, elastic portion 15 attached to lid member 10 moves together with lid member 10. Then, elastic force is no longer applied to a part of the outer circumference of Y-connector 31 that has been held by elastic force applied from elastic portion 15. Therefore, Y-connector 31 can manually be moved. In addition, driven roller 6 also moves together with lid member 10. Pressing force that has been applied from rotation surface 6a of driven roller 6 toward rotation surface 5a of drive roller 5 is released and delivery wire 14 is no longer held, so that delivery wire 104 that has been pressed by the pressing force can manually be moved. As lid member 10 moves to open a part of a periphery of guide groove 17, delivery wire 104 can freely move without being limited to the direction of the longitudinal axis.

Since both of Y-connector 31 and delivery wire 104 can thus manually be moved, delivery wire 104 and Y-connector 31 can integrally be moved while delivery wire 104 is inserted into the through hole in Y-connector 31. Lid member 10 provided in drive device 1 can open a roller portion including drive roller 5 and driven roller 6, the fixing portion where Y-connector 31 is fixed, and guide groove 17. By opening lid member 10, delivery wire 104 inserted in Y-connector 31 and Y-connector 31 can integrally be removed from the fixing portion where Y-connector 31 is fixed. Therefore, even though drive device 1 stops due to occurrence of any such abnormality as power failure, delivery wire 104 and Y-connector 31 can integrally manually be removed.

On the other hand, as lid member 10 is pivoted around hinge 11 serving as the pivot axis from the state that lid member 10 is open, lid member 10 moves in a closing direction. In the cross-sectional view shown in Fig. 3, lid member 10 is closed as lid member 10 is moved clockwise around hinge 11 serving as the pivot center. As the operator holds and operates lever 12, he/she can manually close lid member 10. As protrusion 13 of lever 12 abuts engagement portion 14, lever 12 elastically deforms such that the width of the U shape of lever 12 is narrowed. Protrusion 13 passes by engagement portion 14 as protrusion 13 slides over a surface of engagement portion 14.

As protrusion 13 passes by engagement portion 14, lever 12 elastically deforms such that the width of the U shape is increased and protrusion 13 is engaged with engagement portion 14. When lid member 10 is pushed down to be proximate toward housing 2, movement of driven roller 6 and support member 7 attached to lid member 10 toward housing 2 is impeded by drive roller 5 pivotably supported by motor 3 fixed to housing 2. Therefore, elastic body 8 sandwiched between lid member 10 and support member 7 elastically deforms.

Lid member 10 is pressed by elastic force applied to lid member 10 as reaction by elastic body 8 that has elastically deformed. Protrusion 13 of lever 12 is in intimate contact with engagement portion 14 and lever 12 is pressed against housing 2 by force applied from elastic body 8 toward lid member 10. As lid member 10 is closed, elastic force with which elastic body 8 presses lid member 10 pushes lever 12 toward housing 2. As lever 12 is pressed against housing 2 by elastic force of elastic body 8, lid member 10 is closed.

While lid member 10 is open, Y-connector 31 can be assembled to elastic portion 15 of housing 2 with delivery wire 104 passing through the through hole. When lid member 10 is closed in this state, Y-connector 31 is sandwiched between elastic portion 15 on the housing 2 side and elastic portion 15 on the lid member 10 side. In addition, delivery wire 104 is sandwiched between rotation surface 5a of drive roller 5 and rotation surface 6a of driven roller 6 and arranged in guide groove 17 so as to pass through housing 2 in which guide groove 17 is formed. Delivery wire 104 and Y-connector 31 are supported in drive device 1, by elastic portion 15, between drive roller 5 and driven roller 6 of the roller portion, and by guide groove 17. As lid member 10 is closed, delivery wire 104 and Y-connector 31 can integrally be mounted on the fixing portion of drive device 1.

Lid member 10 can manually be opened and closed by operating lever 12. As lid member 10 is opened as described above, delivery wire 104 and Y-connector 31 can integrally be removed from the fixing portion, and delivery wire 104 and Y-connector 31 can integrally be mounted on the fixing portion by closing lid member 10. Namely, in drive device 1 for delivery wire 104 in the present embodiment, delivery wire 104 and Y-connector 31 can integrally manually be attached to and removed from the fixing portion of drive device 1.

It is noted that delivery wire 104 and Y-connector 31 are attachable to and removable from drive device 1 in such an integrated state that delivery wire 104 is inserted into the through hole in Y-connector 31. Delivery wire 104 and Y-connector 31, however, do not necessarily have to be attached integrally to drive device 1. Namely, in drive device 1 in which the guide portion is provided, delivery wire 104 can reliably be positioned by the guide portion. Therefore, by inserting delivery wire 104 into drive device 1 through the guide portion after lid member 10 is closed and Y-connector 31 is mounted on the fixing portion, delivery wire 104 can also be inserted into the through hole in Y-connector 31. Thus, since it is no longer always necessary to integrally handle delivery wire 104 and Y-connector 31, operability of drive device 1 can further be improved.

Drive device 1 is applied to the insertion device shown in Fig. 1, for inserting delivery wire 104 in blood vessel 132 of human body 131 with delivery wire 104 being inserted into the hollow portion of microcatheter 102 connected to the through hole in Y-connector 31. In this case, even though drive device 1 stops due to occurrence of any such abnormality as power failure, delivery wire 104 and Y-connector 31 can manually integrally be removed. As Y-connector 31 and delivery wire 104 can integrally be removable from drive device 1, positional relation between microcatheter 102 and delivery wire 104 does not change before and after removal. Therefore, treatment can manually be resumed immediately, without substantially changing a position of coil 101 in blood vessel 132 or cerebral aneurysm 133.

A line sensor housing 51 and a light source 81 are attached to Y-connector 31, with delivery wire 104 lying therebetween. A lens 23 and a line sensor 80 are incorporated in line sensor housing 51.

Drive device 1 for a medical linear body described above includes an actuator for moving delivery wire 104 in the direction of the longitudinal axis. The actuator includes motor 3, speed reduction device 9 for reducing a rotation speed of rotational force generated by motor 3 and outputting the resultant rotational force, drive roller 5 carrying out rotational motion as it receives rotational force transmitted from motor 3, and driven roller 6 carrying out rotational motion as drive roller 5 rotates.

Thus, rotation of drive roller 5 for driving delivery wire 104 is caused by motor 3, however, rotational driving force from motor 3 is transmitted to drive roller 5 via speed reduction device 9. As the number of revolutions of rotational driving force generated by motor 3 is decreased by speed reduction device 9, torque can be increased in proportion to a ratio between the number of revolutions of an output shaft of motor 3 and the number of revolutions of an output shaft of speed reduction device 9, that is, a speed reduction ratio. As the number of revolutions is decreased by speed reduction device 9, torque transmitted to drive roller 5 is increased and driving force moving delivery wire 104 in the direction of the longitudinal axis can be increased. Therefore, since desired driving force for delivery wire 104 can be obtained with a small-sized motor, cost for manufacturing drive device 1 can be reduced.

A drive speed of delivery wire 104 is several mm/s. For example, when it is assumed that a moving speed of delivery wire 104 is set to 1 mm/s and a radius of drive roller 5 is set to 10 mm, the number of revolutions of drive roller 5 is approximately 1 rpm (= lmm/s - (10mm × 2π) × 60 s). In order to obtain this number of revolutions of drive roller 5, motor 3 that can rotate at the number of revolutions higher than the number of revolutions of drive roller 5 is used so that the number of revolutions of the output shaft of motor 3 is configured to be decreased by speed reduction device 9 for output to drive roller 5. Then, driving force for delivery wire 104 from drive roller 5 can be increased.

Even though resistance is externally applied to delivery wire 104 by an external load such as friction force at the time when motor 3 drives delivery wire 104, resistance force is expressed as a reciprocal of a speed reduction ratio. If motor 3 rotates at a relatively high speed reduction ratio, for example, approximately from 100 to 1000, resistance force applied to delivery wire 104 with respect to rotational driving force from motor 3 is ignorable. Therefore, delivery wire 104 can be driven at a prescribed speed in a stable manner.

Meanwhile, any facilities capable of feeding elongated delivery wire 104 in a direction of extension thereof may be employed as an actuator for moving delivery wire 104 in the direction of the longitudinal axis, however, an actuator including motor 3 implemented by an electric motor described in the present embodiment is desirable. Drive device 1 including motor 3 implemented by an electric motor and moving delivery wire 104 as it receives rotational force generated by motor 3 could control the number of revolutions of motor 3 based on increase and decrease in voltage to be applied to motor 3.

A moving speed of delivery wire 104 is determined by the number of revolutions of motor 3. For example, in a range of practical use (not higher than insertion force number N), a voltage applied to motor 3 and a moving speed of delivery wire 104 satisfy linear relation. Then, by preparing in advance a relational table between a voltage to be applied to motor 3 and a moving speed of delivery wire 104, a voltage to be applied to motor 3 can be changed in accordance with a moving speed of delivery wire 104 intended by an operator, that is, a rotation speed indicated by drive control device 40.

By thus merely changing a voltage to be applied to motor 3, delivery wire 104 can be moved in the direction of the longitudinal axis at any moving speed, so that a moving speed of delivery wire 104 can be controlled with a simplified configuration. Since such a sensor as an encoder for detecting a rotation speed of drive roller 5 is not required and the number of parts in drive device 1 can be decreased, cost for manufacturing drive device 1 can be reduced and reliability of drive device 1 can be improved. In this case, acting force on delivery wire 104 is detected by measurement device 60 contained in Y-connector 31.

In addition, rotation shaft 4 passes through hole 16a communicating the inside and the outside of small chamber 2b with each other, in which motor 3 and speed reduction device 9 are arranged, and sealing portion 19 in contact with the inner circumferential surface of hole 16a and the outer circumferential surface of rotation shaft 4 is provided in hole 16a.

In a case where delivery wire 104 is implemented by a delivery wire or a guide wire to be inserted in a human body, drive device 1 should be constructed such that cleaning and sterilization of large chamber 2a where drive roller 5 and driven roller 6 for sandwiching delivery wire 104 therebetween and driving the same can readily be carried out. In addition, since physiological saline or a medicine injected through second input port 33 of Y-connector 31 is used during a surgical operation, motor 3 and speed reduction device 9 should be made waterproof. Then, by providing sealing portion 19 to isolate the inside of small chamber 2b from large chamber 2a, introduction of a liquid from large chamber 2a into small chamber 2b can be prevented. Sealing portion 19 arranged between rotation shaft 4 and partition wall 16 can be formed from an elastic material such as a resin material represented by a silicone resin.

Since sealing portion 19 is provided, the outer circumferential surface of rotation shaft 4 comes in contact with sealing portion 19 and slides with respect to the same at the time when rotation shaft 4 rotates, and hence torque required for rotation of rotation shaft 4 increases. In the present embodiment, however, rotational force generated by motor 3 is transmitted from rotation shaft 4 through speed reduction device 9 to drive roller 5 and hence torque transmitted to drive roller 5 increases. Therefore, friction resistance due to slide of rotation shaft 4 with respect to sealing portion 19 is ignorable for motor 3. Therefore, delivery wire 104 can be driven at a prescribed speed in a stable manner.

Referring back to Fig. 1, insertion device 501 includes a foot switch for starting and stopping movement of delivery wire 104 by means of drive device 1 in response to an operation by an operator. The foot switch emits a signal for controlling start-up and stop of drive device 1 in response to an operation to press with a foot. By using the foot switch, the operator who operates microcatheter 102 with his/her hands can perform an ON/OFF operation of drive device 1 without using his/her hands. The foot switch includes insertion foot switch 41 and pull-out foot switch 46. Insertion foot switch 41 is connected to drive control device 40 through line 42. Pull-out foot switch 46 is connected to drive control device 40 through line 47.

By an operation to press insertion foot switch 41 with a foot, a contained microswitch is pressed and drive device 1 is actuated so as to move delivery wire 104 in a direction in which delivery wire 104 is inserted in blood vessel 132. Specifically, drive roller 5 rotates and thus delivery wire 104 is driven in an advancing direction, that is, in a direction of insertion into microcatheter 102. Thus, an insertion operation for inserting coil 101 in cerebral aneurysm 133 is performed.

By an operation to press pull-out foot switch 46 with a foot, the contained microswitch is pressed and drive device 1 is actuated so as to move delivery wire 104 in a direction in which delivery wire 104 is pulled out of blood vessel 132. Specifically, drive roller 5 rotates in a reverse direction and thus delivery wire 104 is driven in a backing-off direction, that is, in a direction of pull-out from microcatheter 102. Thus, a pull-out operation for pulling delivery wire 104 having coil 101 attached at the tip end out of blood vessel 132 is performed.

By moving a foot away from insertion foot switch 41 or pull-out foot switch 46, pressing of the contained microswitch is released by elastic force of a return spring. Consequently, since driving force no longer acts on delivery wire 104, delivery wire 104 stops.

A doctor who provides coil embolization treatment with the use of this insertion device can perform an operation at holding portion 105 around the inlet port of Y-connector 111 while securing Y-connector 111 in which microcatheter 102 has been inserted with his/her left hand and holding microcatheter 102 with his/her right hand. In addition, as the same doctor operates insertion foot switch 41 and pull-out foot switch 46 with his/her foot, delivery wire 104 can be inserted in microcatheter 102 and coil 101 can be inserted in cerebral aneurysm 133. Namely, one doctor can perform an operation of catheter 102 and an operation of delivery wire 104.

Therefore, by using this insertion device, one doctor can provide coil embolization treatment for embolizing cerebral aneurysm 133 by leaving coil 101 in cerebral aneurysm 133. Since one doctor can provide coil embolization treatment that has conventionally been provided by two doctors, it is not necessary for two doctors to provide treatment in cooperation with each other and hence stress imposed on the doctor involved with cooperation can be mitigated.

Here, microcatheter 102 is manually operated as the doctor holds microcatheter 102 at holding portion 105, as in the conventional example. On the other hand, drive device 1 for moving delivery wire 104 is provided and delivery wire 104 is driven by drive device 1. Start-up and stop of drive device 1 is controlled by an operation of insertion foot switch 41 or pull-out foot switch 46. The doctor who operates microcatheter 102 with his/her hand operates the foot switch with his/her foot.

Since the insertion device for delivery wire 104 allowing one doctor to provide coil embolization treatment can thus be realized with a simplified construction, cost for manufacturing the insertion device can be reduced and reliability of the insertion device can be improved. Since insertion foot switch 41 operated at the time when delivery wire 104 is to be advanced and pull-out foot switch 46 operated at the time when delivery wire 104 is to be backed off are separately provided, an erroneous operation at the time of insertion and pull-out of delivery wire 104 is less likely. Therefore, reliability of the insertion device can further be improved.

Drive control device 40 shown in Fig. 1 further includes a speed instruction portion for controlling a speed of delivery wire 104 moved by drive device 1 in response to an operation by an operator. More specifically, an insertion speed instruction portion 43 is electrically connected through a line 44 and a pull-out speed instruction portion 48 is electrically connected through a line 49. Volume switches 45 and 50 capable of adjusting a moving speed at which drive device 1 moves delivery wire 104 are attached to insertion speed instruction portion 43 and pull-out speed instruction portion 48, respectively.

As volume switch 45, 50 provided at the speed instruction portion (insertion speed instruction portion 43 and pull-out speed instruction portion 48) is operated, a speed of insertion or a speed of pull-out of delivery wire 104 can be increased and decreased. A doctor who provides coil embolization treatment alone holds Y-connector 111 with his/her left hand when he/she inserts microcatheter 102 into Y-connector 111. When this doctor desires to increase or decrease a speed of insertion or a speed of pull-out of delivery wire 104, he/she operates volume switch 45, 50 of the speed instruction portion (that is, insertion speed instruction portion 43 or pull-out speed instruction portion 48) with his/her right hand. Thus, the moving speed of delivery wire 104 in the direction of the longitudinal axis can be controlled.

By allowing the speed instruction portion to vary a moving speed of delivery wire 104, delivery wire 104 can continuously be inserted in blood vessel 132 during coil embolization treatment. Namely, coil 101 attached to the tip end of delivery wire 104 can continuously be inserted in aneurysm 133. Therefore, increase in insertion resistance of coil 101 as coil 101 comes in contact with a wall portion of aneurysm 133 (an aneurysm wall) in a static state to thereby generate static friction force between coil 101 and the aneurysm wall can be suppressed. Namely, when coil 101 is inserted in aneurysm 133, fluctuation in compressive force in the direction of the longitudinal axis applied to delivery wire 104 can be suppressed.

In addition, a moving speed of delivery wire 104 can finely be adjusted by means of the speed instruction portion. Therefore, in a case where a delicate operation is required, for example, a case where coil 101 is left in aneurysm 133, a moving speed of delivery wire 104 can be decreased to improve operation reliability.

Measurement device 60 is incorporated in Y-connector 31. Namely, measurement device 60 is integrated with Y-connector 31.

Fig. 6 is an external view showing a construction of a main body of a measurement device according to Embodiment 1 of the present invention.

Referring to Fig. 6, measurement device 60 includes a main body 52, in which a through hole 53 into which delivery wire 104 having flexibility is inserted is formed. Fig. 6 shows a state that measurement device 60 is set on a floor surface. Though not shown, line sensor 80 which will be described later is arranged on a floor surface side of measurement device 60, and light source 81 which will be described later is arranged on a ceiling surface side of measurement device 60. Main body 52 is, for example, a transparent body, and it is formed of a substance through which light can pass.

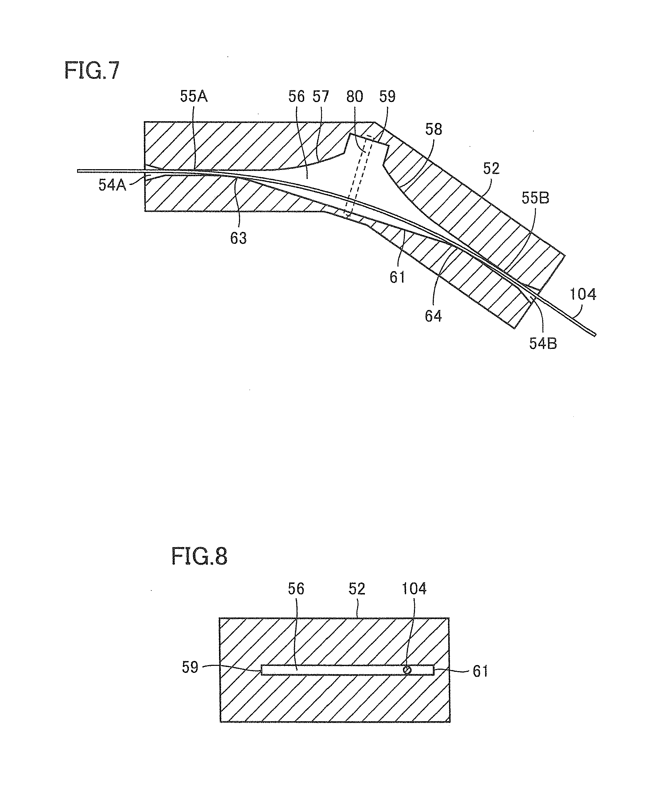

Fig. 7 is a cross-sectional view showing a cross-section along the line VII-VII in Fig. 6. Fig. 8 is a cross-sectional view showing a cross-section along the line VIII-VIII in Fig. 6.

Referring to Fig. 7, through hole 53 has tapered input/output ports 54A, 54B as a first end portion and a second end portion respectively, in order to improve insertion performance by making an entrance and an exit into which delivery wire 104 is inserted greater. Through hole 53 has restraint portions 55A, 55B provided between input/output ports 54A, 54B, for restricting movement of delivery wire 104 in a direction other than the direction of the longitudinal axis of delivery wire 104. In restraint portions 55A, 55B, through hole 53 is slightly greater in diameter than delivery wire 104 (for example, 105% to 120% of a diameter of delivery wire 104). In addition, a length of through hole 53 along the direction of the longitudinal axis of delivery wire 104 is at least several times as great as the diameter of delivery wire 104. Therefore, in restraint portions 55A, 55B, an operation of delivery wire 104 is restrained in a direction other than the direction of the longitudinal axis.

Through hole 53 is formed to allow bending of delivery wire 104 in an arc shape in through hole 53 and variation in degree of bending of delivery wire 104 in accordance with compressive force and pulling force applied to delivery wire 104. Namely, through hole 53 is formed such that delivery wire 104 is bent in an arc shape in a detection portion 56 within through hole 53 while compressive force and pulling force in the direction of the longitudinal axis are not applied to delivery wire 104. Further, through hole 53 is formed such that the degree of bending of delivery wire 104 increases when compressive force is applied to delivery wire 104 as compared with when compressive force and pulling force are not applied to delivery wire 104 and the degree of bending of delivery wire 104 decreases when pulling force is applied to delivery wire 104 as compared with when compressive force and pulling force are not applied to delivery wire 104.

According to such a construction, even when compressive force and pulling force in the direction of the longitudinal axis applied to delivery wire 104 are very small, compressive force and pulling force can accurately be detected.

Main body 52 defines a direction of bending of delivery wire 104 within through hole 53 when compressive force and pulling force in the direction of the longitudinal axis are applied to delivery wire 104. Namely, through hole 53 is bent between two restraint portions 55A, 55B, and as delivery wire 104 is inserted into through hole 53, delivery wire 104 is in a bent shape. In addition, through hole 53 is formed to make up such a detection portion 56 that inner walls 57, 58 are distant from an inner wall 61 and a diameter of through hole 53 increases between two restraint portions 55A, 55B therein.

Restraint portions 55A, 55B are provided in input/output port 54A and input/output port 54B respectively, in a manner adjacent thereto, and each has an opening area S1 when it is cut in a plane orthogonal to a direction of insertion of delivery wire 104. Detection portion 56 is provided between restraint portion 55A and restraint portion 55B and it has an opening area S2 greater than opening area S1 when it is cut in a plane orthogonal to the direction of insertion of delivery wire 104. Then, detection portion 56 is formed such that delivery wire 104 is bent in an arc shape, and expanded in a direction of an inner periphery and a direction of an outer periphery of delivery wire 104 bent in the arc shape.

Detection portion 56 is formed as inner walls 57, 58 located on an outer side of bending of delivery wire 104 extend. Inner wall 61 is formed as a plane. In detection portion 56, an operation of delivery wire 104 in a direction parallel to the sheet surface of Fig. 7 is not restricted. Delivery wire 104 passes through the inside of main body 52 while it is bent in detection portion 56.

In input/output ports 54A, 54B and detection portion 56, a height of through hole 53 in a direction perpendicular to the sheet surface of Fig. 7 is slightly greater than the diameter of delivery wire 104 (for example, 105% to 120% of the diameter of delivery wire 104), and an operation of delivery wire 104 in a direction perpendicular to the sheet surface of Fig. 7 is restrained. Namely, in input/output ports 54A, 54B and detection portion 56, a cross-sectional shape of through hole 53 in a cross-section perpendicular to the direction of the longitudinal axis of delivery wire 104 is rectangular. Thus, a direction of bending of delivery wire 104 within through hole 53 is defined, and delivery wire 104 is positioned such that a height of a crest of a bending portion of delivery wire 104 when compressive force and pulling force in the direction of the longitudinal axis are applied to delivery wire 104 (that is, a maximum value of a distance from inner wall 61 to delivery wire 104) is determined.

Line sensor 80 is arranged in detection portion 56 so as to extend across the cross-section of through hole 53 in detection portion 56. Line sensor 80 is arranged such that it extends across the inside of detection portion 56 from inner wall 61 of through hole 53 to the inside of a recess portion 59 making up an inner wall of through hole 53 opposed to inner wall 61, which will be described later. Line sensor 80 is arranged along a trace of peaks of the crest of bending that are formed as delivery wire 104 is bent in the arc shape when compressive force and pulling force in the direction of the longitudinal axis are applied to delivery wire 104 in detection portion 56.

In addition, as shown in Fig. 7, boundary portions 63, 64 between inner wall 61 in detection portion 56 and restraint portions 55A, 55B are formed in a curved surface shape convex toward the inside of through hole 53. Inner walls 57, 58 are formed in a curved surface shape convex toward the inside of through hole 53. Inner wall 57 is formed in a curved surface shape as being in contact with the inner wall of through hole 53 in restraint portion 55A, while inner wall 58 is formed in a curved surface shape as being in contact with the inner wall of through hole 53 in restraint portion 55B. Thus, bending of delivery wire 104 accompanying plastic deformation can be prevented. In addition, recess portion 59 is formed between inner wall 57 and inner wall 58 in detection portion 56. Recess portion 59 is formed as the inner wall of through hole 53 is recessed toward an external side of main body 52 such that the inner wall of through hole 53 between inner wall 57 and inner wall 58 in detection portion 56 is more distant from inner wall 61.

The wall portion of detection portion 56 is shaped such that inner walls 57, 58 each in a curved surface shape convex toward the inside of through hole 53 and recess portion 59 are combined. Owing to such a shape of detection portion 56, when delivery wire 104 is bent as compressive force in the direction of the longitudinal axis is applied to delivery wire 104 in detection portion 56, delivery wire 104 can be bent along the inner wall (that is, inner wall 57 and inner wall 58) of through hole 53 located outside bending of delivery wire 104. Further, a part of delivery wire 104 can be bent away from inner wall 57 and inner wall 58. Furthermore, as compressive force increases, a distance between contact points, that are points where delivery wire 104 is away from inner walls 57, 58, decreases.

Therefore, buckling of delivery wire 104 in detection portion 56 can be suppressed. Namely, even in a case where delivery wire 104 small in buckling load is employed, delivery wire 104 can be bent in detection portion 56 without buckling and hence a degree of bending of delivery wire 104 can accurately be detected. By converting the detected degree of bending, compressive force in the direction of the longitudinal axis applied to delivery wire 104 can be measured.

In addition, since recess portion 59 is formed in detection portion 56, compressive force applied to delivery wire 104 can accurately be measured over a wide range. Namely, by detecting a height of a crest of bending of delivery wire 104 in detection portion 56, compressive force applied to delivery wire 104 is measured. Here, unless a peak of the bending portion of delivery wire 104 located within detection portion 56, that is, a point on delivery wire 104 located within detection portion 56 most distant from inner wall 61, is in contact with the inner wall of detection portion 56, compressive force applied to delivery wire 104 can be measured. If recess portion 59 is formed, greater compressive force in the direction of the longitudinal axis will be required in order to bring the peak of the bending portion of delivery wire 104 in contact with the inner wall of detection portion 56. Therefore, a range of measurement of compressive force applied to delivery wire 104 can be widened.

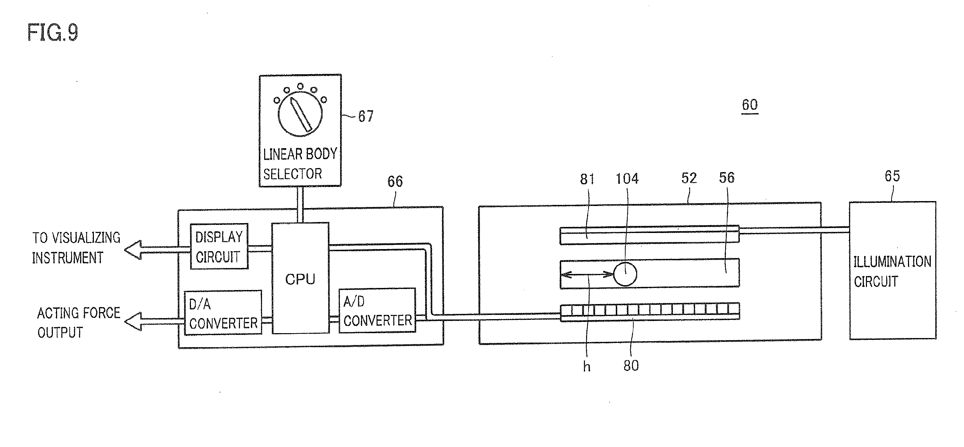

Fig. 9 is a schematic diagram showing an overall configuration of the measurement device.

Referring to Fig. 9, measurement device 60 further includes light source 81 emitting light, line sensor 80 serving as a light receiver for receiving light emitted from light source 81, an illumination circuit 65 causing light source 81 to emit light, and a conversion circuit 66.

Line sensor 80 is a one-dimensional optical array sensor having a plurality of light-receiving elements receiving light arranged in line.

An optical path extending from light source 81 to line sensor 80 in the inside of main body 52 is composed of a light-transmissive material through which light used for detection passes.

Light source 81 and line sensor 80 are arranged with detection portion 56 lying therebetween, so as to oppose to each other with delivery wire 104 lying therebetween.

Line sensor 80 is arranged along a direction of height of the crest of bending of delivery wire 104, that is, a direction of movement of the peak of the crest of bending of delivery wire 104 when compressive force and pulling force in the direction of the longitudinal axis are applied to delivery wire 104. Line sensor 80 is arranged in a direction perpendicular to a direction of extension of inner wall 61 and arranged to be orthogonal to delivery wire 104 at the peak of the crest of bending. Line sensor 80 detects a degree of bending of delivery wire 104 by measuring a height h of the crest of bending of delivery wire 104.

A degree of bending of delivery wire 104 is detected based on shadow of delivery wire 104 projected on line sensor 80. Namely, when line sensor 80 receives light emitted by light source 81 and when delivery wire 104 is located over a certain light-receiving element of line sensor 80, the light emitted by light source 81 is cut off by delivery wire 104. Then, a quantity of light received by that light-receiving element decreases. A position of that light-receiving element corresponds to the degree of bending of delivery wire 104.

The construction is not limited to such a construction that the light receiver arranged at a position opposed to the light source receives transmitting light, and the light source and the light receiver may be arranged side by side and such a reflector as a mirror for reflecting light emitted by the light source may be set at a position opposed to the light source. In this case, as the light receiver receives reflection light reflected by the reflector, of the light emitted by the light source, a degree of bending of a linear body can similarly be detected. Alternatively, a degree of bending of the linear body can also be detected with a two-dimensional array sensor in which a plurality of light-receiving elements are arranged, for example, in matrix on a plane, instead of the one-dimensional array sensor such as a line sensor. In addition, since a degree of bending of the linear body should only be detected, for example, a non-contact distance sensor for detecting a height of a crest of bending, a position sensor for detecting a position of the linear body, or the like can also be employed.

Illumination circuit 65 and conversion circuit 66 are provided outside main body 52. Illumination circuit 65 causes light source 81 to emit light. Conversion circuit 66 converts a degree of bending of delivery wire 104 detected based on a quantity of light received by line sensor 80 with respect to the quantity of light emitted by light source 81 into a signal indicating compressive force and pulling force in the direction of the longitudinal axis applied to delivery wire 104 and outputs the signal. It is noted that conversion circuit 66 may have an amplifier circuit for amplifying an output from line sensor 80.

Conversion circuit 66 converts a degree of bending of delivery wire 104 into a signal indicating compressive force and pulling force applied to delivery wire 104 based on prescribed correlation between the degree of bending of delivery wire 104 and compressive force and pulling force applied to delivery wire 104 and outputs the resultant signal. It is noted that, in order to have an image of delivery wire 104 appropriately formed on line sensor 80, such an optical element as a lens, a slit, and a filter for cutting off external light may be set in the present optical system.

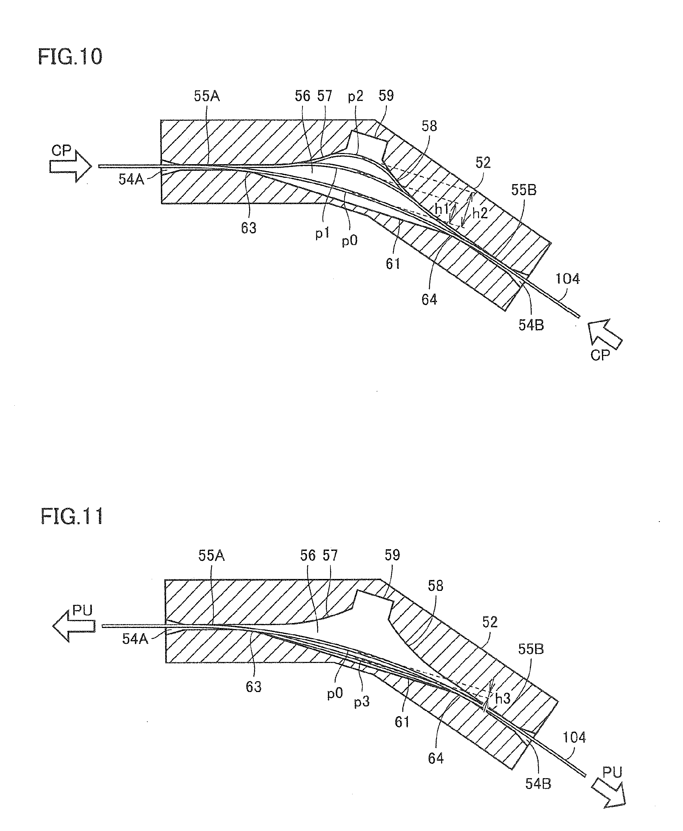

Fig. 10 is a diagram showing such a state that compressive force is applied to delivery wire 104 and delivery wire 104 is bent in main body 52 in the cross-sectional view in Fig. 7.

Referring to Fig. 10, when compressive force CP in the direction of the longitudinal axis is applied to delivery wire 104, a degree of bending of delivery wire 104 increases. With increase in degree of bending of delivery wire 104, a height of the crest of bending becomes greater.

In Fig. 10, a state of delivery wire 104 while compressive force and pulling force are not applied to delivery wire 104 is indicated with p0. In state p0, delivery wire 104 is bent in an arc shape.

When compressive force CP is applied to delivery wire 104, delivery wire 104 is further bent relative to state p0 and the height of the crest of bending increases by h1 as compared with state p0 (a state p1).

When compressive force CP greater than in state p1 is applied to delivery wire 104, delivery wire 104 is further bent relative to state p1, the height of the crest of bending further increases as compared with state p1, and the height increases by h2 (h2 > h1) as compared with state p0 (a state p2).

Fig. 11 is a diagram showing such a state that pulling force is applied to delivery wire 104 and delivery wire 104 is bent in main body 52 in the cross-sectional view in Fig. 7.

Referring to Fig. 11, when pulling force PU in the direction of the longitudinal axis is applied to delivery wire 104, a degree of bending of delivery wire 104 decreases. With decrease in degree of bending of delivery wire 104, the height of the crest of bending becomes smaller.

In Fig. 11, a state of delivery wire 104 while compressive force and pulling force are not applied to delivery wire 104 is indicated with p0. In state p0, delivery wire 104 is bent in an arc shape.

When pulling force PU is applied to delivery wire 104, a degree of bending of delivery wire 104 decreases relative to state p0 and the height of the crest of bending decreases by h3 as compared with state p0 (a state p3).

When the degree of bending of delivery wire 104 increases as compared with the degree of bending of delivery wire 104 while compressive force and pulling force are not applied to delivery wire 104, conversion circuit 66 converts the detected degree of bending into a signal indicating compressive force applied to delivery wire 104. Alternatively, when the degree of bending of delivery wire 104 decreases as compared with the degree of bending of delivery wire 104 while compressive force and pulling force are not applied to delivery wire 104, conversion circuit 66 converts the detected degree of bending into a signal indicating pulling force applied to delivery wire 104.

For example, line sensor 80 detects a position of the bending portion of delivery wire 104 within through hole 53. When delivery wire 104 is displaced from a reference position of delivery wire 104 while compressive force and pulling force are not applied to delivery wire 104 toward an outer periphery, conversion circuit 66 converts an amount of displacement from the reference position into a signal indicating compressive force applied to delivery wire 104, and when delivery wire 104 is displaced from the reference position toward an inner periphery, conversion circuit 66 converts an amount of displacement from the reference position into a signal indicating pulling force applied to delivery wire 104.

When line sensor 80 receives light emitted by light source 81 arranged at a position opposed to line sensor 80 with detection portion 56 lying therebetween and when delivery wire 104 is located over a certain light-receiving element of the plurality of light-receiving elements in line sensor 80, the light emitted by light source 81 is cut off by delivery wire 104. Then, a quantity of light received by that light-receiving element decreases. By detecting a position of that light-receiving element, a position of delivery wire 104 can be specified and increase and decrease in height of the crest of bending of delivery wire 104, that is, a degree of bending of delivery wire 104, can be detected.

Conversion circuit 66 converts a height of the crest of bending of delivery wire 104 into a signal indicating compressive force and pulling force applied to delivery wire 104 based on prescribed correlation between the height of the crest of bending of delivery wire 104 detected by line sensor 80 and compressive force and pulling force applied to delivery wire 104 (for example, a set of compressive force or pulling force of certain magnitude and a height of the crest of bending corresponding thereto) and outputs the signal. Compressive force and pulling force applied in the direction of the longitudinal axis of delivery wire 104 can thus be measured.

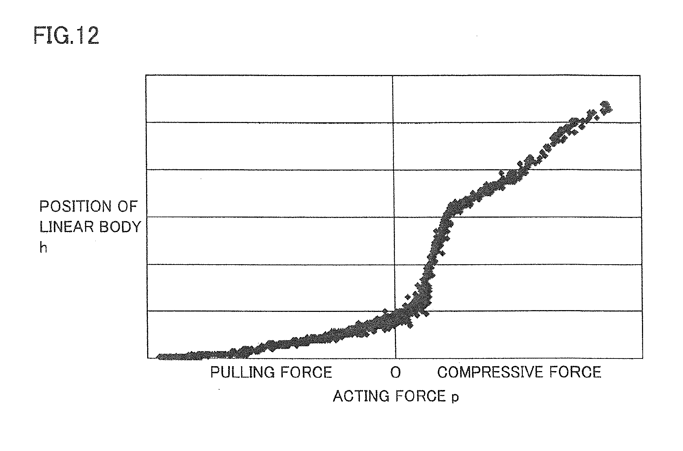

Fig. 12 is a diagram showing correlation between force applied to the linear body and a position of the linear body detected by a line sensor.

Fig. 12 shows results of measurement of force applied to the linear body and a position of the linear body detected by the line sensor. In Fig. 12, the abscissa represents acting force p in the direction of the longitudinal axis applied to delivery wire 104, and the ordinate represents height h of the crest of bending of delivery wire 104.

Conversion circuit 66 stores correlation shown in Fig. 12 and converts an electric signal received from line sensor 80 into a signal indicating compressive force and pulling force applied to delivery wire 104 based on this correlation. It is noted that conversion circuit 66 may store correlation shown in Fig. 12 as it is or store an equation approximating correlation shown in Fig. 12.

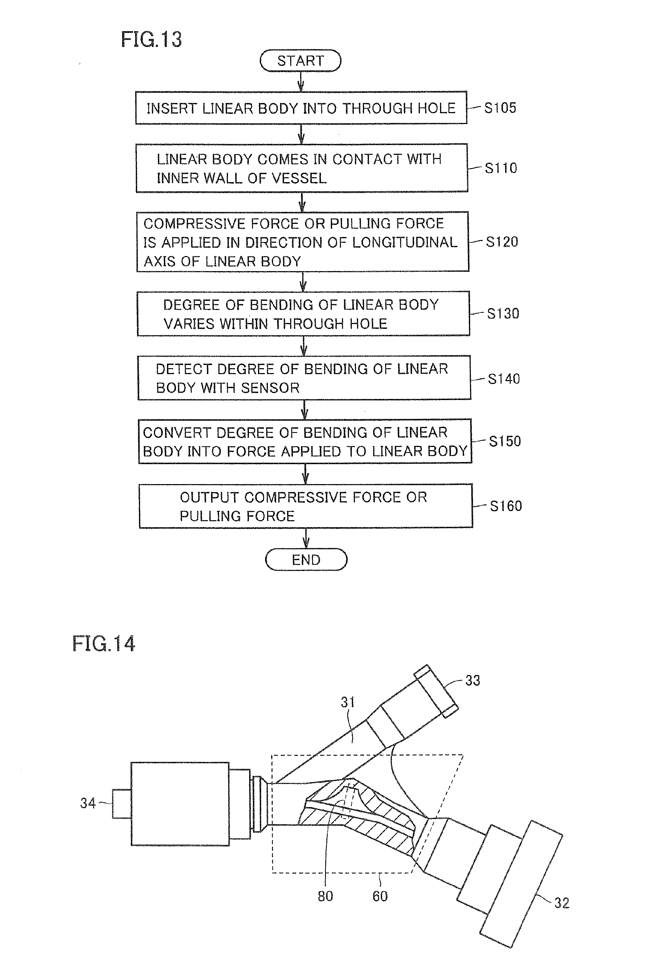

Fig. 13 is a diagram showing a procedure of a measurement method according to Embodiment 1 of the present invention.

Referring to Fig. 13, initially, in a step (S105), delivery wire 104 is inserted into through hole 53 of main body 52. Namely, delivery wire 104 is inserted into through hole 53 formed to allow bending of delivery wire 104 in an arc shape therein and variation in degree of bending of delivery wire 104 in accordance with compressive force and pulling force.

Then, in a step (S110), a tip end of delivery wire 104 comes in contact with an inner wall of a vessel as a result of insertion of delivery wire 104 in the vessel and operation of the delivery wire from the outside of the vessel.

Then, in a step (S120), when force is applied in the direction of the longitudinal axis of delivery wire 104 from the outside of the vessel in order to further insert or pull out delivery wire 104, an operation of delivery wire 104 is restricted depending on whether the tip end of delivery wire 104 is in contact with the inner wall of the vessel, delivery wire 104 is caught in the vessel in the body, or the like. Therefore, compressive force or pulling force is applied in the direction of the longitudinal axis of delivery wire 104.

Then, in a step (S130), as a result of acting compressive force or pulling force, a degree of bending of delivery wire 104 varies in detection portion 56 within through hole 53.

Then, in a step (S140), line sensor 80 detects the degree of bending of delivery wire 104.

Then, in a step (S150), conversion circuit 66 converts the degree of bending of delivery wire 104 detected in the step (S140) into a signal indicating compressive force or pulling force applied to delivery wire 104 based on prescribed correlation between the degree of bending of delivery wire 104 and compressive force and pulling force applied to delivery wire 104.

Then, in a step (S160), the signal indicating compressive force or pulling force, which results from conversion from the degree of bending of delivery wire 104, is output.

As above, with the measurement device and the measurement method according to Embodiment 1 of the present invention, when compressive force or pulling force in the direction of the longitudinal axis is applied to delivery wire 104, line sensor 80 detects a degree of bending of delivery wire 104. Then, conversion circuit 66 converts the detected degree of bending of delivery wire 104 into a signal indicating compressive force or pulling force in the direction of the longitudinal axis applied to delivery wire 104. In addition, since line sensor 80 is provided at a position where delivery wire 104 is operated, which is located outside the vessel in which delivery wire 104 is inserted, so as to measure compressive force and pulling force in the direction of the longitudinal axis applied to delivery wire 104, compressive force and pulling force can quantitatively be measured also for extremely thin delivery wire 104 in which it is difficult to provide a pressure sensor at the tip end.

Namely, with the measurement device and the measurement method according to Embodiment 1 of the present invention, force for inserting the linear body and force for pulling out the linear body can both be measured. In addition, since the construction is simple, incorporation into medical equipment is easy and hence a warning can be issued when excessive load is applied to a human body and a linear body serving as a treatment device in an operation for inserting and pulling out the linear body to be inserted in a human body. Therefore, with the measurement device and the measurement method according to Embodiment 1 of the present invention, the linear body can satisfactorily be operated with a simplified construction.

If a shape and a material, that is, a Young's modulus, of delivery wire 104 are different, a degree of bending of delivery wire 104 at the time when the same force is applied will be different. Therefore, when a plurality of delivery wires 104 different in shape and material are used, correlation between degrees of bending of various linear bodies different in shape and material and force in the direction of the longitudinal axis applied to the linear bodies is determined in advance and stored in conversion circuit 66. Then, measurement device 60 includes also a linear body selector 67 shown in Fig. 9, so that linear body selector 67 selects which correlation is to be used, in accordance with a linear body that is used. Thus, as the same measurement device is applicable to delivery wires 104 of various shapes and materials, linear bodies that have been used for various different applications so far can be used without modification, and cost effectiveness is achieved.

Fig. 14 is a diagram showing a construction of a Y-connector according to Embodiment 1 of the present invention.

Referring to Fig. 14, Y-connector 31 includes measurement device 60, input ports 32 and 33, and output port 34.

The Y-connector serves for inserting a linear body in a catheter in using the catheter, and it has input port 32 which is an insertion port of the linear body and input port 33 which is a physiological saline injection port for injecting physiological saline into the catheter.

Measurement device 60 is incorporated in a passage communicating input port 32 and output port 34 with each other within Y-connector 31. Delivery wire 104 is, for example, a linear medical appliance such as a guide wire and a catheter inserted in a vessel in a body such as a blood vessel and a ureter, or a wire having an embolus coil attached at a tip end for embolizing an aneurysm. Delivery wire 104 is guided to a destination in the body through an operation from an input port 32 side.

By measuring compressive force and pulling force in the direction of the longitudinal axis applied to delivery wire 104, reaction force against compressive force and pulling force, that is, load applied by delivery wire 104 to the vessel in the body, can be measured. Namely, contact of the tip end of the medical appliance with the inner wall of the vessel, delivery wire 104 being caught in the vessel in the body, or the like can be sensed, and excessive load can be prevented from acting on the vessel in the body.

In addition, since measurement device 60 is incorporated in Y-connector 31, delivery wire 104 can be operated through input port 32 of Y-connector 31 while a medicine can be injected through input port 33. For example, physiological saline for reducing friction between the catheter and the guide wire can be injected through input port 33. In addition, after the catheter inserted in the blood vessel is guided from the outside of a human body to the destination, a contrast medium can be injected through input port 33 so that the contrast medium can reach the destination in the body.

In inserting a coil and a delivery wire in a catheter in coil embolization, a Y-connector is employed. Therefore, integration of the Y-connector with measurement device 60 is very useful because usability so far of the Y-connector remains unchanged. Since the Y-connector is disposable, such a structure that the Y-connector and a sensor housing can be separated from each other may be adopted.

In measurement device 60 for measuring compressive force applied to delivery wire 104, line sensor 80 representing an optical sensor is employed. By employing the optical sensor, as shown in Fig. 9, a light-transmissive material such as a transparent resin material can be arranged between light source 81 and detection portion 56 and between line sensor 80 and detection portion 56. Since such sensor parts as light source 81 and line sensor 80 can be contained in main body 52, the sensor parts are not exposed to detection portion 56. Therefore, the sensor parts can be prevented from coming in contact with such a liquid as physiological saline injected though another input port 33 of Y-connector 31. Thus, since it is not necessary to take into consideration occurrence of such defects as failure of the sensor parts due to contact with a liquid, cleaning and sterilization of Y-connector 31 incorporating measurement device 60 can readily be achieved.

As a notification device for notifying an operator of compressive force and pulling force measured with measurement device 60, representatively, a visualizing instrument displaying compressive force and pulling force measured with measurement device 60 in a numeric value, on a meter, or in a graph, and an auralizing instrument converting compressive force and pulling force into voice and sound corresponding thereto are employed. The insertion device in the present embodiment can include any one of the visualizing instrument and the auralizing instrument, or can also include both of them.

As shown in Fig. 1, a sensor output control device 90 for controlling an output from the visualizing instrument and an output from the auralizing instrument is electrically connected through a line 91 to measurement device 60. Sensor output control device 90 notifies an operator of compressive force and pulling force applied to delivery wire 104 through light or sound, and changes luminance of light, a wavelength of light, a frequency of light, the number of light beams intermittently emitted per a prescribed time period, sound volume, a wavelength of sound, a frequency of sound, or the number of sounds intermittently emitted per a prescribed time period in accordance with magnitude of compressive force and pulling force measured with measurement device 60.

Fig. 1 exemplifies a display 93 serving as the visualizing instrument for displaying a numeric value showing compressive force and pulling force applied to delivery wire 104 as converted from a voltage output from measurement device 60.

In addition, Fig. 1 exemplifies an auralizing instrument varying an acoustic effect, for example, emitting a warning alarm from a speaker 92, when a voltage output from measurement device 60 exceeds a prescribed threshold value, that is, when compressive force and pulling force applied to delivery wire 104 each exceed a prescribed threshold value.

During coil embolization treatment, a doctor pays attention to an X-ray fluoroscopic image of aneurysm 133 and coil 101. Therefore, it is convenient, by using the auralizing instrument, to present to the doctor, compressive force and pulling force applied to delivery wire 104 through sound when delivery wire 104 is driven by drive device 1.

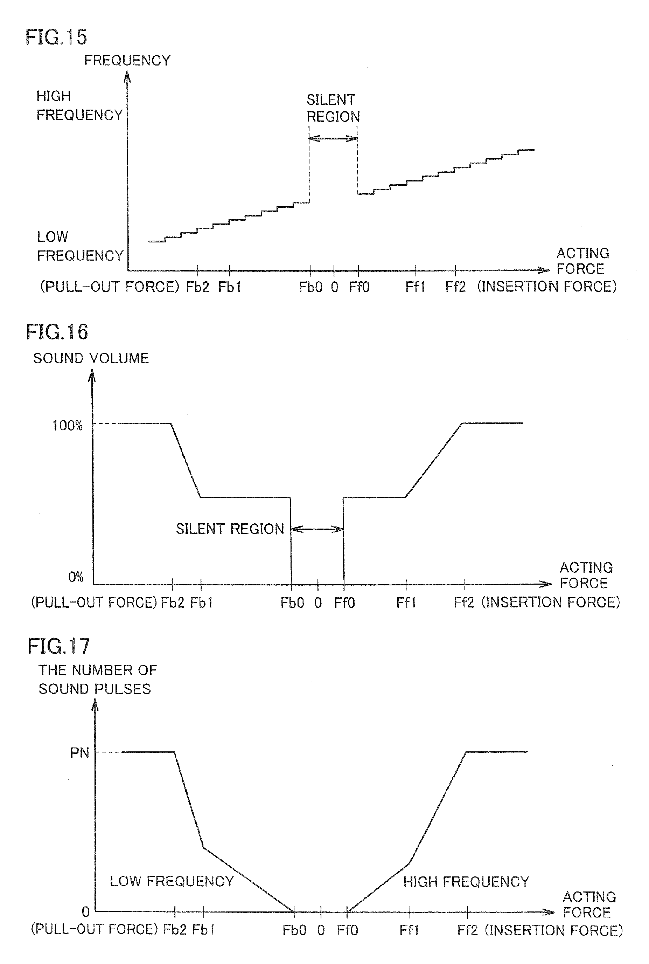

Fig. 15 is a diagram showing acoustic control carried out by the sensor output control device in the insertion device according to Embodiment 1 of the present invention. In Fig. 15, the abscissa represents force applied to delivery wire 104 (acting force) and the ordinate represents a frequency of a warning alarm. Here, compressive force (insertion force) is defined as positive acting force and pulling force (pull-out force) is defined as negative acting force.

For example, when compressive force and pulling force applied to delivery wire 104 become excessively great, that is, when compressive force applied to delivery wire 104 exceeds a threshold value Ff0 or when pulling force applied to delivery wire 104 is more negative than a threshold value Fb0, sensor output control device 90 controls speaker 92 to generate a warning alarm and vary stepwise a tone of the warning alarm (low-pitch sound and high-pitch sound, intermittent sound and continuous sound, etc.) with increase in compressive force and pulling force.

In such a case that acting force is thus presented to a doctor through an acoustic effect, by varying a frequency stepwise in accordance with magnitude of acting force, abnormality can be notified more reliably than by continuously varying a frequency depending on magnitude of acting force. In addition, by tuning a step of a frequency to a musical scale, abnormality can further reliably be notified.

In addition, when acting force on delivery wire 104 is equal to or smaller than threshold value Ff0 of insertion force and equal to or more positive than threshold value Fb0 of pulling force, sensor output control device 90 does not generate a warning alarm because no large force is applied to delivery wire 104.

The fact that compressive force and pulling force applied to delivery wire 104 have become excessively great can be presented to a doctor also through change in visual effect by making use of light. In a case where light is used instead of sound, the ordinate in Fig. 15 should only be replaced with a wavelength of light. Namely, for example, when compressive force and pulling force applied to delivery wire 104 become excessively great, that is, when compressive force applied to delivery wire 104 exceeds threshold value Ff0 or when pulling force applied to delivery wire 104 is more negative than threshold value Fb0, sensor output control device 90 controls a warning indicator 94 shown in Fig. 1 to emit warning light and vary stepwise a wavelength of warning light with increase in compressive force and pulling force.

For example, a color of light emitted from the indicator may be changed around a prescribed threshold value. With increase in compressive force and pulling force, a color of light emitted from the indicator and a blink rate may be varied stepwise. Alternatively, speaker 92 and warning indicator 94 may be used together. By abruptly changing a visual effect or an acoustic effect around the threshold value, a doctor's attention can reliably be attracted, which is further effective.

Fig. 16 is a diagram showing acoustic control carried out by the sensor output control device in the insertion device according to Embodiment 1 of the present invention. In Fig. 16, the abscissa represents force applied to delivery wire 104 (acting force) and the ordinate represents volume of a warning alarm (sound volume). Here, insertion force is defined as positive acting force and pull-out force is defined as negative acting force.

For example, when compressive force and pulling force applied to delivery wire 104 become excessively great, that is, when compressive force applied to delivery wire 104 exceeds threshold value Ff0 or when pulling force applied to delivery wire 104 is more negative than threshold value Fb0, sensor output control device 90 controls speaker 92 to generate a warning alarm and increase sound volume of the warning alarm with increase in compressive force and pulling force.

More specifically, when acting force on delivery wire 104 is equal to or greater than threshold value Ff0 of insertion force and smaller than a threshold value Ff1 thereof or when acting force on delivery wire 104 is more positive than a threshold value Fb1 of pull-out force and equal to or more negative than threshold value Fb0 thereof, sensor output control device 90 sets sound volume of the warning alarm to a prescribed value between 0% and 100% of a reference value. Alternatively, when acting force on delivery wire 104 is equal to or greater than threshold value Ff1 of insertion force and smaller than a threshold value Ff2 thereof or when acting force on delivery wire 104 is more positive than a threshold value Fb2 of pull-out force and equal to or more negative than threshold value Fb1 thereof, sensor output control device 90 continuously changes sound volume of the warning alarm in accordance with compressive force and pulling force applied to delivery wire 104. Even when acting force on delivery wire 104 is equal to or greater than threshold value Ff2 of insertion force or when acting force on delivery wire 104 is more negative than threshold value Fb2 of pull-out force, sensor output control device 90 maintains the sound volume of the warning alarm constant at 100% of the reference value.

When acting force on delivery wire 104 is equal to or smaller than threshold value Ff0 of insertion force and equal to or more positive than threshold value Fb0 of pulling force, sensor output control device 90 does not generate a warning alarm because no large force is applied to delivery wire 104.

In a case where light is used instead of sound, the ordinate in Fig. 16 should only be replaced with luminance of light. Namely, for example, when compressive force and pulling force applied to delivery wire 104 become excessively great, that is, when compressive force applied to delivery wire 104 exceeds threshold value Ff0 or when pulling force applied to delivery wire 104 is more negative than threshold value Fb0, sensor output control device 90 controls warning indicator 94 to emit warning light and change luminance of the warning light with increase in compressive force and pulling force, similarly to the warning alarm as above.

Fig. 17 is a diagram showing acoustic control carried out by the sensor output control device in the insertion device according to Embodiment 1 of the present invention. In Fig. 17, the abscissa represents force applied to delivery wire 104 (acting force) and the ordinate represents the number of sound pulses generated in a prescribed time period. Here, insertion force is defined as positive acting force and pull-out force is defined as negative acting force.

For example, when compressive force and pulling force applied to delivery wire 104 become excessively great, that is, when compressive force applied to delivery wire 104 exceeds threshold value Ff0 or when pulling force applied to delivery wire 104 is more negative than threshold value Fb0, sensor output control device 90 controls speaker 92 to increase the number of pulses of the warning alarm, that is, the number of warning alarms intermittently generated in a prescribed time period.

More specifically, when acting force on delivery wire 104 is equal to or greater than threshold value Ff0 of insertion force and smaller than threshold value Ff1 thereof or when acting force on delivery wire 104 is more positive than threshold value Fb1 of pull-out force and equal to or more negative than threshold value Fb0 thereof, sensor output control device 90 continuously changes the number of pulses of the warning alarm in accordance with compressive force and pulling force applied to delivery wire 104. Alternatively, when acting force on delivery wire 104 is equal to or greater than threshold value Ff1 of insertion force and smaller than threshold value Ff2 thereof or when acting force on delivery wire 104 is more positive than threshold value Fb2 of pull-out force and equal to or more negative than threshold value Fb1 thereof, sensor output control device 90 makes further greater an extent of variation in the number of pulses of the warning alarm in accordance with compressive force and pulling force applied to delivery wire 104. Even when acting force on delivery wire 104 is equal to or greater than threshold value Ff2 of insertion force or when acting force on delivery wire 104 is equal to or more negative than threshold value Fb2 of pull-out force, sensor output control device 90 maintains the number of pulses of the warning alarm constant at a prescribed value PN.

When acting force on delivery wire 104 is equal to or smaller than threshold value Ff0 of insertion force and equal to or more positive than threshold value Fb0 of pulling force, sensor output control device 90 does not generate a warning alarm because no large force is applied to delivery wire 104.

When compressive force is applied to delivery wire 104, sensor output control device 90 sets a frequency of a sound pulse to be high, and when pulling force is applied to delivery wire 104, it sets a frequency of a sound pulse to be low. Thus, a doctor can recognize a direction of acting force.

In a case where light is used instead of sound, the ordinate in Fig. 17 should only be replaced with the number of light pulses generated in a prescribed time period and a frequency should only be replaced with a wavelength of light. Namely, for example, when compressive force and pulling force applied to delivery wire 104 become excessively great, that is, when compressive force applied to delivery wire 104 exceeds threshold value Ff0 or when pulling force applied to delivery wire 104 is more negative than threshold value Fb0, sensor output control device 90 controls warning indicator 94 to increase the number of pulses of warning light, that is, the number of warning light beams intermittently generated in a prescribed time period (the number of times of blinking). When compressive force is applied to delivery wire 104, sensor output control device 90 sets a wavelength of a light pulse to be long, and when pulling force is applied to delivery wire 104, it sets a wavelength of a light pulse to be short. Thus, a doctor can recognize a direction of acting force.

By thus using the visualizing instrument or the auralizing instrument, when compressive force and pulling force applied to delivery wire 104 become excessively great, a doctor can reliably recognize that fact through a warning alarm or turn-on of an indicator. Therefore, even when one doctor operates both of microcatheter 102 and delivery wire 104 in coil embolization treatment, insertion force applied to delivery wire 104 becoming excessively great and excessive load being applied to cerebral aneurysm 133 can readily be suppressed.

In addition, the insertion device according to the present embodiment includes drive device 1 for moving delivery wire 104 in the direction of the longitudinal axis and foot switches 41, 46 for emitting a signal for controlling start-up and stop of drive device 1. Thus, a doctor who operates with his/her hand microcatheter 102 through which delivery wire 104 has been inserted can operate foot switch 41, 46 with his/her foot to be able to move delivery wire 104, and hence ease in operating the insertion device is improved. Therefore, one doctor can realize an operation of microcatheter 102 and delivery wire 104.

Moreover, the insertion device according to the present embodiment includes the measurement device for measuring compressive force and pulling force in the direction of the longitudinal axis applied to delivery wire 104 and the notification device for notifying an operator of compressive force and pulling force measured with the measurement device. Thus, increase in compressive force and pulling force applied to cerebral aneurysm 133 by coil 101 at the tip end of delivery wire 104 can reliably be recognized by a doctor, so that excessive load can be prevented from acting on cerebral aneurysm 133. Namely, even when one doctor operates microcatheter 102 and delivery wire 104, coil 101 at the tip end of delivery wire 104 can reliably be prevented from applying excessive load to cerebral aneurysm 133, so that ease in operating the insertion device can further be improved.

Further, drive device 1 for moving delivery wire 104 in the direction of the longitudinal axis includes speed reduction device 9 for decreasing a rotation speed of rotational force generated by motor 3 and outputting the resultant rotational force. Since torque transmitted to drive roller 5 can thus be increased in proportion to a speed reduction ratio, driving force for delivery wire 104 can be increased even in a case where small-sized motor 3 is used, and reduction in cost for manufacturing drive device 1 and in size thereof can be realized. Since the number of revolutions of motor 3 is controlled by a voltage applied to motor 3 and a moving speed of delivery wire 104 can be adjusted only by varying a voltage, cost for manufacturing drive device 1 can further be reduced and reliability of drive device 1 can be improved.

Furthermore, motor 3 and speed reduction device 9 are contained in a case (that is, in small chamber 2b) formed by housing 2 and partition wall 16, hole 16a is formed in partition wall 16, and rotation shaft 4 for transmitting rotational force from speed reduction device 9 to drive roller 5 passes through hole 16a. On the inner circumferential surface of hole 16a, sealing portion 19 being in contact with the outer circumferential surface which is the rotation surface of rotation shaft 4 and cutting off the inside of small chamber 2b from the outside is provided. Thus, since the internal space in large chamber 2a where drive roller 5 and driven roller 6 are arranged and the internal space in small chamber 2b can be isolated from each other, introduction of a liquid from large chamber 2a into small chamber 2b can be prevented. Therefore, motor 3 and speed reduction device 9 can be made waterproof, and hence cleaning and sterilization of the inside of large chamber 2a can readily be achieved.

Rotation surfaces 5a and 6a of drive roller 5 and driven roller 6 respectively are formed of an elastic material. In addition, feed groove 5b is formed in rotation surface 5a of drive roller 5 and delivery wire 104 is arranged in feed groove 5b. Thus, delivery wire 104 is correctly set between drive roller 5 and driven roller 6 and not sandwiched in other portions when lid member 10 is closed. Moreover, since friction force generated between rotation surface 5a, 6a and delivery wire 104 sandwiched between rotation surfaces 5a and 6a can be increased, slipping of delivery wire 104 with respect to rotation surface 5a, 6a during movement of delivery wire 104 can be suppressed.

In the description so far, the insertion device for inserting delivery wire 104 having coil 101 for embolization treatment for embolizing cerebral aneurysm 133 attached at the tip end into blood vessel 132 of human body 131 has been described. The insertion device according to the present invention should only be an insertion device for inserting a medical linear body, which is a linear medical appliance having flexibility, in a vessel in a body such as a blood vessel, a ureter, a bronchus, an alimentary canal, or a lymph vessel, and guiding the medical linear body to a destination through an operation from the outside of the body. For example, a medical linear body may be a catheter or a guide wire.

Another embodiment of the present invention will now be described with reference to the drawings. It is noted that the same or corresponding elements in the drawings have the same reference characters allotted and description thereof will not be repeated.

### <Embodiment 2>

The present embodiment relates to an insertion device different in shape of a through hole from the insertion device according to Embodiment 1. The insertion device is similar to the insertion device according to Embodiment 1 except for contents which will be described below.

A measurement device according to Embodiment 2 of the present invention is suitable for cerebral aneurysm coil embolization, which is a treatment method for preventing rupture of a cerebral aneurysm representing a cause of subarachnoid hemorrhage. The present treatment is treatment for densely filling an aneurysm with a coil with a catheter serving as a guide tube.

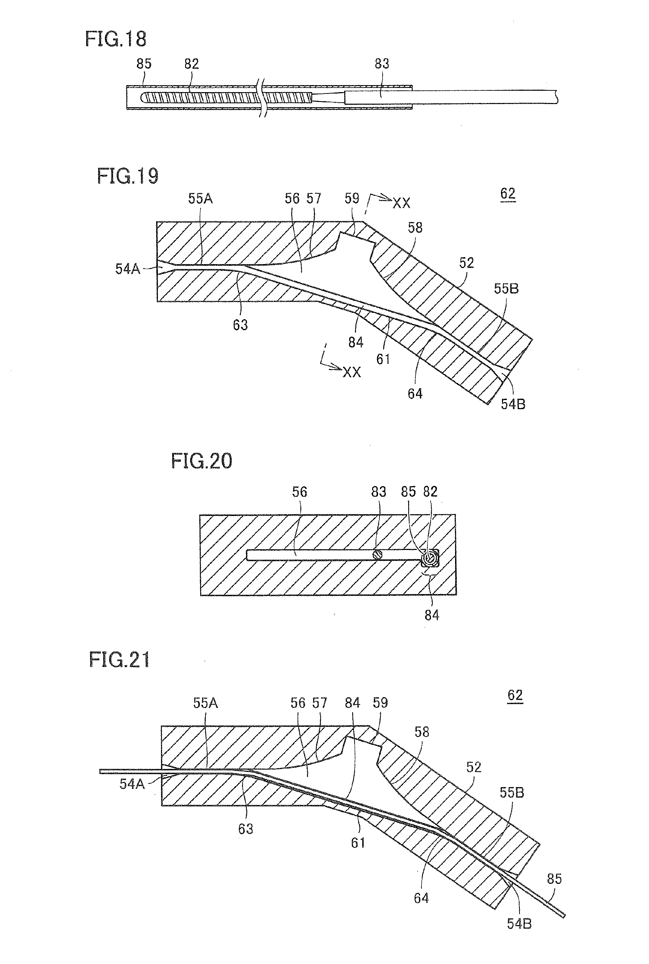

Fig. 18 is a diagram showing a construction of a coil for embolization of a cerebral aneurysm with a coil.

Referring to Fig. 18, a coil for embolization of a cerebral aneurysm with a coil includes a coil portion 82 used for embolization and a delivery wire portion 83 for feeding coil portion 82.

A coil for embolization of a cerebral aneurysm with a coil has a spiral shape or a cage shape in conformity with a size of an aneurysm, and before use, it extends straight as shown in Fig. 18, for easy insertion in a catheter.

An operator operates delivery wire portion 83 so as to fill the aneurysm with coil portion 82. After the operator inserts coil portion 82, he/she changes the coil portion with a coil portion different in size, and after the operator leaves coil portion 82, he/she pulls out delivery wire portion 83. In particular, since coil portion 82 is flexible, forced pull-out leads to extension of the coil portion. Therefore, it is important to perform an operation for pulling out coil portion 82 without applying excessive pulling force to coil portion 82.

In addition, in coil embolization, an aneurysm susceptible to rupture is densely filled with coil portion 82. Therefore, in order not to apply excessive force to the aneurysm, it is also important to measure insertion force in inserting delivery wire portion 83, that is, compressive force applied to the linear body.

Here, since coil portion 82 is flexible, it is accommodated in a sheath 85 before use. In a case where coil portion 82 is inserted in a human body through a catheter, sheath 85 and an inlet of the catheter are aligned with each other such that coil portion 82 is not out of place. Then, delivery wire portion 83 is pushed forward to move coil portion 82 into the catheter. Then, when coil portion 82 completely entered the catheter, sheath 85 is removed from delivery wire portion 83 so that delivery wire portion 83 is operated.

Since sheath 85 is greater in diameter than delivery wire portion 83, sheath 85 cannot be inserted in main body 52 of measurement device 60 according to Embodiment 1 of the present invention. If sheath 85 is removed and coil portion 82 is inserted in measurement device 60, coil portion 82 will spread in detection portion 56.

Then, in the measurement device according to Embodiment 2 of the present invention, a through hole is formed as follows.

Fig. 19 is a cross-sectional view showing a construction of the measurement device according to Embodiment 2 of the present invention. Fig. 19 corresponds to Fig. 7 in Embodiment 1 of the present invention. Fig. 20 is a cross-sectional view showing a cross-section along the line XX-XX in Fig. 19. Fig. 20 corresponds to Fig. 8 in Embodiment 1 of the present invention. Fig. 21 is a diagram showing such a state that sheath 85 is inserted into main body 52 in the cross-sectional view in Fig. 19.

Referring to Fig. 19, through hole 53 in a measurement device 62 includes a sheath groove 84 which is a groove dedicated for passage of sheath 85. More specifically, sheath groove 84 is provided between restraint portion 55A and restraint portion 55B and connected to detection portion 56, and a length thereof in a direction at a right angle to a direction of insertion of delivery wire 104 and a direction of radius of delivery wire 104 bent in an arc shape is greater than detection portion 56.

Therefore, as shown in Fig. 20, sheath 85 can pass through only sheath groove 84 between restraint portions 55A and 55B. Then, passage of sheath 85 through main body 52 is as shown in Fig. 21.

Measurement device 62 and a catheter are connected to each other, and sheath 85 and a mouth of the catheter are aligned with each other. Then, after delivery wire portion 83 is pushed to cause coil portion 82 to completely enter the catheter, sheath 85 is pulled out of main body 52. Then, delivery wire portion 83 remains in main body 52. Here, delivery wire portion 83 bends owing to its rigidity. Since delivery wire portion 83 is thinner than sheath 85, it moves from sheath groove 84 to detection portion 56 and it is bent as in measurement device 60 as shown in Fig. 7.

A portion of through hole 53 other than sheath groove 84 has a width slightly greater than a diameter of delivery wire portion 83. Therefore, as in measurement device 60, a position of a linear body in detection portion 56 is uniquely determined in correspondence with acting force, and sheath 85 can pass through without deteriorating accuracy in detection of acting force.

In addition, measurement device 62 is provided with sheath groove 84 in a portion of detection portion 56 where delivery wire portion 83 does not pass through. Namely, sheath groove 84 is provided opposite to the direction of bending of delivery wire 104. Sheath groove 84 is connected to the end portion of detection portion 56 on the inner periphery side of delivery wire 104 bent in an arc shape. According to such a construction, sheath groove 84 can readily be made.

As the construction and the operation are otherwise the same as those of the insertion device according to Embodiment 1, detailed description will not be repeated here.

Another embodiment of the present invention will now be described with reference to the drawings. It is noted that the same or corresponding elements in the drawings have the same reference characters allotted and description thereof will not be repeated.

### <Embodiment 3>

The present embodiment relates to an insertion device different in control of a speed of a delivery wire from the insertion device according to Embodiment 1. The insertion device is similar to the insertion device according to Embodiment 1 except for contents which will be described below.

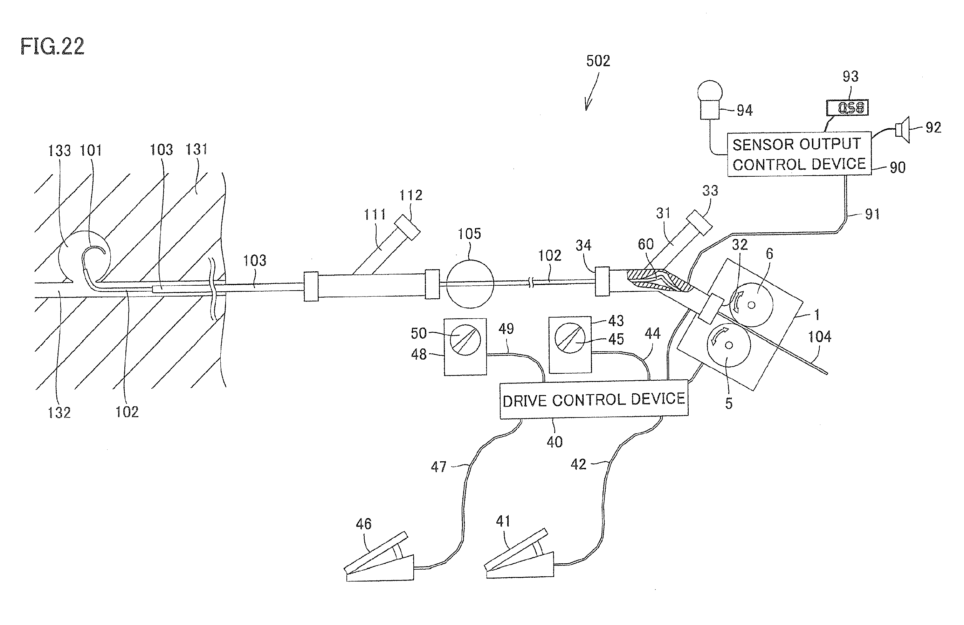

Fig. 22 is a diagram showing a construction of an insertion device according to Embodiment 3 of the present invention.

Referring to Fig. 22, in an insertion device 502, drive control device 40 controls a speed for drive of delivery wire 104 by drive device 1, that is, a moving speed of delivery wire 104, based on compressive force and pulling force in the direction of the longitudinal axis applied to delivery wire 104, that is measured with measurement device 60.

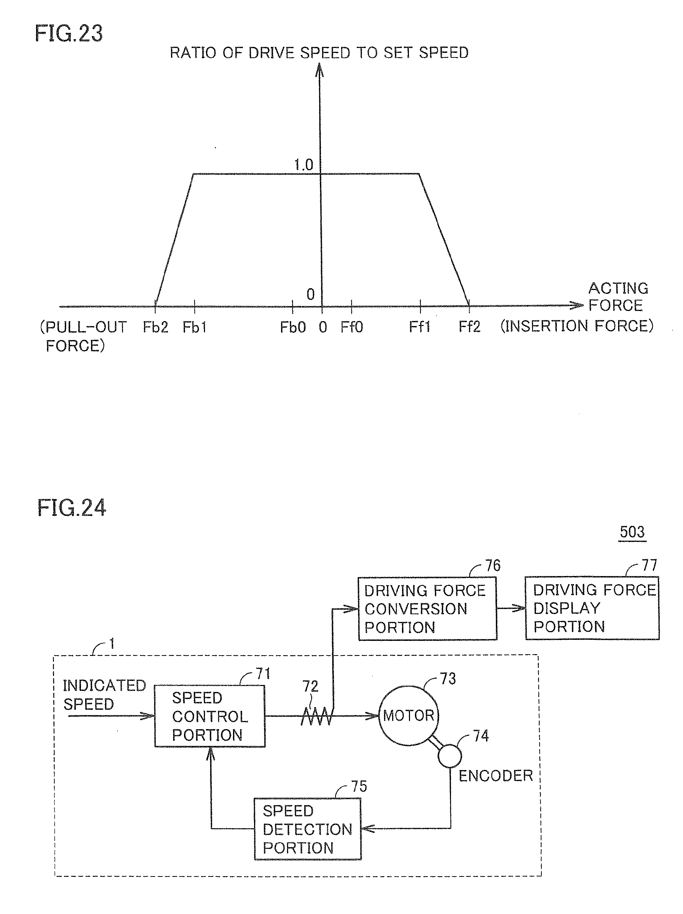

Fig. 23 is a diagram showing drive speed control carried out by the insertion device according to Embodiment 3 of the present invention. In Fig. 23, the abscissa represents force applied to delivery wire 104 (acting force) and the ordinate represents a ratio of an actual drive speed to a set speed of delivery wire 104. Here, insertion force is defined as positive acting force and pull-out force is defined as negative acting force.

When excessive insertion force and pull-out force are applied to delivery wire 104, drive control device 40 carries out control such that a drive speed for delivery wire 104 is lowered from a set speed.

When compressive force measured with measurement device 60 exceeds a prescribed threshold value or when pulling force measured with measurement device 60 exceeds a prescribed threshold value, drive control device 40 controls drive device 1 so as to decrease a moving speed of delivery wire 104 in accordance with an amount of excess of compressive force and pulling force over each threshold value or stop movement of delivery wire 104.

Specifically, when acting force on delivery wire 104 exceeds threshold value Ff1 of insertion force, drive control device 40 continuously decreases an insertion speed from the set speed. Then, when insertion force for delivery wire 104 exceeds threshold value Ff2, drive control device 40 stops drive of delivery wire 104.

Alternatively, when acting force on delivery wire 104 is more negative than threshold value Fb1 of pulling force, drive control device 40 continuously decreases a pull-out speed from the set speed. Then, when pulling force for delivery wire 104 is more negative than threshold value Fb2, drive control device 40 stops drive of delivery wire 104.

Therefore, with the insertion device according to Embodiment 3 of the present invention, excessive insertion force and pull-out force can further reliably be prevented from acting on a human body, a treatment device, and a medical linear body.

As the construction and the operation are otherwise the same as those of the insertion device according to Embodiment 1, detailed description will not be repeated here.

Another embodiment of the present invention will now be described with reference to the drawings. It is noted that the same or corresponding elements in the drawings have the same reference characters allotted and description thereof will not be repeated.

### <Embodiment 4>

The present embodiment relates to an insertion device different in a method of measuring compressive force and pulling force applied to a linear body, from the insertion device according to Embodiment 1. The insertion device is similar to the insertion device according to Embodiment 1 except for contents which will be described below.

Fig. 24 is a diagram showing a configuration of a drive device in the insertion device according to Embodiment 4 of the present invention.

Referring to Fig. 24, an insertion device 503 includes drive device 1, a driving force conversion portion 76, and a driving force display portion 77. Drive device 1 includes a speed control portion 71, a current detector 72, a motor 73, an encoder (a rotation speed detection portion) 74, and a speed detection portion 75. Motor 73 corresponds to motor 3 in insertion device 501.

By eliminating speed reduction device 9 or lowering a speed reduction ratio of speed reduction device 9 in drive device 1 according to Embodiment 1 of the present invention, driving force for delivery wire 104 can be detected as a load of motor 73. Thus, force applied to delivery wire 104 can be estimated from a drive current for motor 73. In this case, since output from motor 73 affects load on delivery wire 104, a rotation encoder should be provided for the motor in order to control a speed of motor 73.

Motor 73 generates rotational force for moving delivery wire 104 in the direction of the longitudinal axis based on a supplied drive current. Encoder 74 detects a rotation speed of motor 73. Speed detection portion 75 detects a moving speed of delivery wire 104 in the direction of the longitudinal axis based on the rotation speed detected by encoder 74. Speed control portion 71 generates a drive current such that a moving speed detected by linear body speed detection portion 75 is equal to an indicated speed received from drive control device 40. Current detector 72 detects a drive current.

Driving force conversion portion 76 measures compressive force and pulling force applied to delivery wire 104 based on a drive current detected by current detector 72. Namely, driving force conversion portion 76 converts a drive current detected by current detector 72 into driving force. Driving force conversion portion 76 takes the place of measurement device 60 in insertion device 501.

Driving force display portion 77 displays driving force obtained by conversion by driving force conversion portion 76. It is noted that insertion device 503 may be configured to emit sound and light based on driving force obtained by conversion by driving force conversion portion 76, similarly to insertion device 501.

As the construction and the operation are otherwise the same as those of the insertion device according to Embodiment 1, detailed description will not be repeated here.

Another embodiment of the present invention will now be described with reference to the drawings. It is noted that the same or corresponding elements in the drawings have the same reference characters allotted and description thereof will not be repeated.

### <Embodiment 5>

The present embodiment relates to a recording system for recording a result of measurement in an insertion device. The present embodiment is similar to the insertion device according to Embodiment 1 except for contents which will be described below.

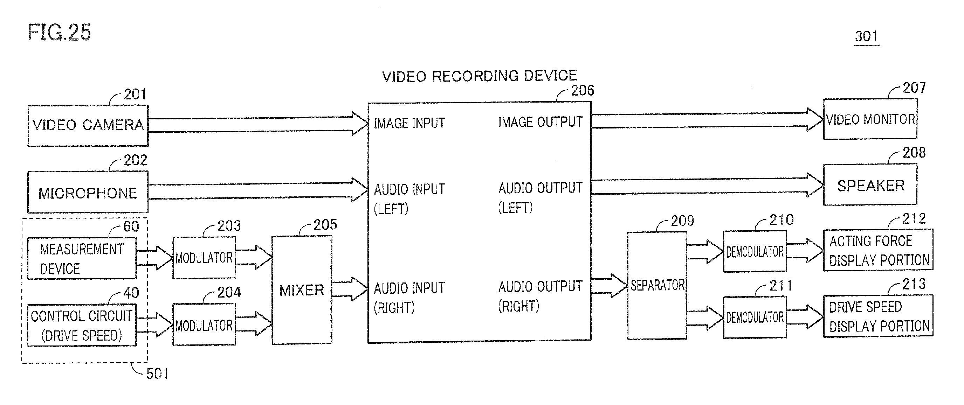

Fig. 25 is a diagram showing a configuration of a linear body operation recording system according to Embodiment 5 of the present invention.

Referring to Fig. 25, a linear body operation recording system 301 includes a video camera 201, a microphone 202, insertion device 501, modulators 203, 204, a mixer 205, a video recording device 206, a video monitor 207, a speaker 208, a separator 209, demodulators 210, 211, an acting force display portion 212, and a drive speed display portion 213.

Video camera 201 picks up an X-ray fluoroscopic image of a tip end portion of delivery wire 104 inserted in a vessel in a body and the surroundings thereof and generates a video signal. The video signal generated by video camera 201 is provided to an image input terminal of video recording device 206. Microphone 202 converts into an audio signal, voice and sound during a surgical operation using delivery wire 104. The audio signal generated by microphone 202 is provided to one terminal of a stereo audio input terminal pair of video recording device 206 (in Fig. 25, a left audio input terminal).

Measurement device 60 in insertion device 501 measures compressive force and pulling force in the direction of the longitudinal axis of delivery wire 104 and outputs a signal indicating the measured value. Modulator 203 modulates and outputs a signal received from measurement device 60. Drive control device 40 in insertion device 501 outputs a signal indicating a drive speed for delivery wire 104. Modulator 204 modulates and outputs a signal received from drive control device 40. Mixer 205 mixes an output signal from modulator 203 and an output signal from modulator 204 with each other. A signal obtained by mixing by mixer 205 is provided to the other terminal of the stereo audio input terminal pair of video recording device 206 (in Fig. 25, a right audio input terminal). A signal obtained by mixing by mixer 205 is recorded in a DVD recorder or on a video recording medium such as a video tape together with an X-ray fluoroscopic image from video camera 201 and voice and sound from microphone 202. Here, video recording device 206 records an X-ray fluoroscopic image from video camera 201, voice and sound from microphone 202, compressive force and pulling force in the direction of the longitudinal axis of delivery wire 104, and a drive speed for delivery wire 104, in synchronization with one another, that is, records them temporally in correspondence with one another.

During reproduction, video monitor 207 displays an X-ray fluoroscopic image based on a video signal received from video recording device 206. Speaker 208 emits sound based on an audio signal received from video recording device 206. Separator 209 is implemented, for example, by a filter, and it separates a mixed signal received from video recording device 206. Demodulator 210 demodulates a signal separated by separator 209 and outputs a signal indicating acting force. Demodulator 211 demodulates a signal separated by separator 209 and outputs a signal indicating a drive speed. Acting force display portion 212 displays acting force based on a signal received from demodulator 211. Drive speed display portion 213 displays a drive speed based on a signal received from demodulator 210.

Though linear body operation recording system 301 makes recording by mixing a signal indicating acting force and a drive speed with an audio signal, it may make recording by mixing the same with a video signal.

As the configuration and the operation are otherwise the same as those of the insertion device according to Embodiment 1, detailed description will not be repeated here.

Another embodiment of the present invention will now be described with reference to the drawings. It is noted that the same or corresponding elements in the drawings have the same reference characters allotted and description thereof will not be repeated.

### <Embodiment 6>

The present embodiment relates to an insertion device different in an assembly structure from the insertion device according to Embodiment 1. The insertion device is similar to the insertion device according to Embodiment 1 except for contents which will be described below.

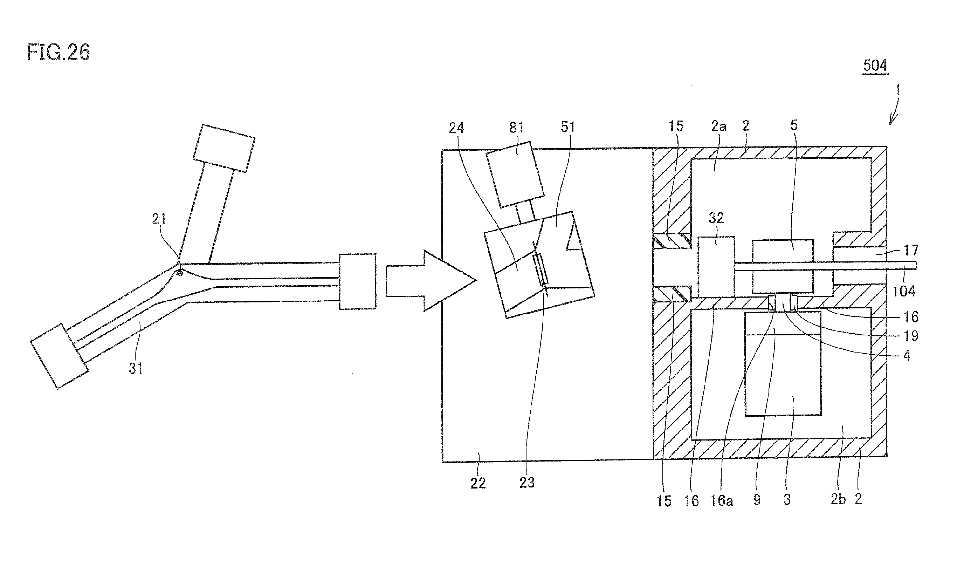

Fig. 26 is a diagram showing a separated structure of a Y-connector, a measurement device, and a drive device in the insertion device according to Embodiment 6 of the present invention.

Referring to Fig. 26, in an insertion device 504, Y-connector 31 is attachable to and removable from line sensor 80. Line sensor housing 51 and light source 81 are integrated with drive device 1, for example, with a base 22 interposed therebetween. Light source 81 is movable, and after Y-connector 31 is fitted into a fitting groove 24 in line sensor housing 51, light source 81 is displaced to a prescribed position. Thus, line sensor housing 51 and light source 81 are opposed to each other, with delivery wire 104 lying therebetween. Lens 23 and line sensor 80 are incorporated in line sensor housing 51.

A marker 21 is provided in Y-connector 31, a position of marker 21 is detected in line sensor housing 51, and a result of measurement of acting force is corrected based on this position. Thus, conversion in acting force measurement based on flexure of delivery wire 104 can be prevented from erring due to a fitting position.

As the construction and the operation are otherwise the same as those of the insertion device according to Embodiment 1, detailed description will not be repeated here.

Another embodiment of the present invention will now be described with reference to the drawings. It is noted that the same or corresponding elements in the drawings have the same reference characters allotted and description thereof will not be repeated.

### <Embodiment 7>

The present embodiment relates to an insertion device different in a light source provided in a line sensor housing from the insertion device according to Embodiment 6. The insertion device is similar to the insertion device according to Embodiment 6 except for contents which will be described below.

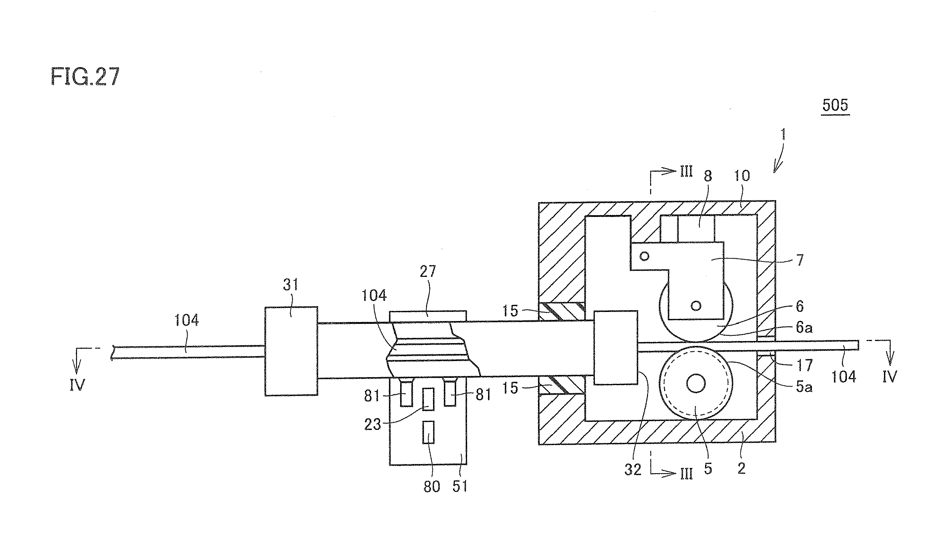

Fig. 27 is a diagram showing a separated structure of a Y-connector, a measurement device, and a drive device in an insertion device according to Embodiment 7 of the present invention.

Referring to Fig. 27, in an insertion device 505, a reflector 27 is set in Y-connector 31, and light source 81 is set in housing 51 as aligned with line sensor 80. Light output from light source 81 is reflected by reflector 27, and line sensor 80 receives this reflected light.

In insertion device 505, Y-connector 31 and line sensor housing 51 can be separated from each other. By integrating reflector 27 and Y-connector 31 with each other in insertion device 504 in which a movable light source is employed, such drudgery as displacement of light source 81 to a prescribed position after Y-connector 31 is attached to line sensor housing 51 can be eliminated.

As the construction and the operation are otherwise the same as those of the insertion device according to Embodiment 6, detailed description will not be repeated here.

Another embodiment of the present invention will now be described with reference to the drawings. It is noted that the same or corresponding elements in the drawings have the same reference characters allotted and description thereof will not be repeated.

### <Embodiment 8>

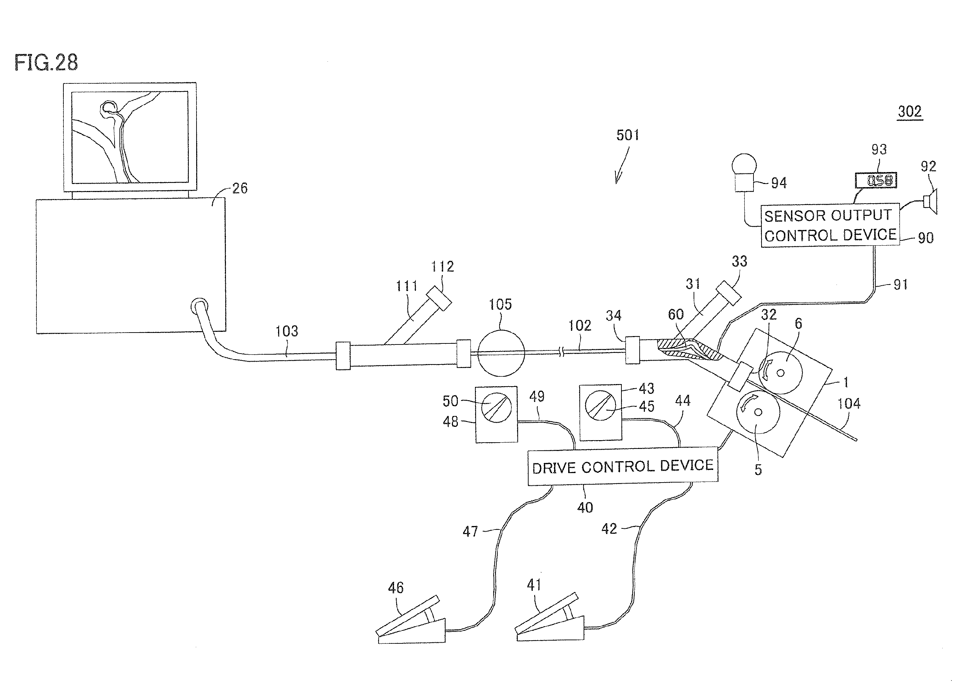

Fig. 28 is a diagram showing a construction of a training device according to Embodiment 8 of the present invention.

Referring to Fig. 28, a training device 302 includes insertion device 501 and a simulator 26.

As an operator holding delivery wire 104 applies compressive force or pulling force to delivery wire 104 in order to advance delivery wire 104 into simulator 26 or pull the same out of simulator 26, that compressive force or pulling force is displayed on display 93.

Simulator 26 simulates a human body and displays an image equivalent to a perspective image of a vessel in a human body. An operator in training of a medical device operates delivery wire 104 while viewing an image displayed on simulator 26. Simulator 26 varies insertion resistance and pull-out resistance of inserted delivery wire 104. When compressive force and pulling force applied to delivery wire 104 are each equal to or greater than a prescribed threshold value, a warning alarm is output from speaker 92.

Though insertion device 501 and simulator 26 are separate from each other in Fig. 28, the construction may be such that measurement device 501 and simulator 26 are integrated. Further, the construction may be such that compressive force and pulling force applied to delivery wire 104 are additionally displayed on a simulated perspective image displayed on simulator 26, instead of display 93.

According to such a construction, manipulation of a skilled operator can be quantified and manipulation of a less experienced operator can quickly be improved. In addition, manipulation by the operator can be recorded together with a perspective image, as records during surgical operation.

As the construction and the operation are otherwise the same as those of the insertion device according to Embodiment 1, detailed description will not be repeated here.

It should be understood that the embodiments disclosed herein are illustrative and non-restrictive in every respect. The scope of the present invention is defined by the terms of the claims, rather than the description above, and is intended to include any modifications within the scope and meaning equivalent to the terms of the claims.

### REFERENCE SIGNS LIST

1 drive device; 2 housing; 2a large chamber; 2b small chamber; 3 motor; 4 rotation shaft; 5a rotation surface; 5b groove; 5 drive roller; 6a rotation surface; 6 driven roller; 7 support member; 8 elastic body; 9 speed reduction device; 10 lid member; 11 hinge; 12 lever; 13 protrusion; 14 engagement portion; 15 elastic portion; 16a hole; 16 partition wall; 17 guide groove; 18 projection portion; 19 sealing portion; 21 marker; 22 base; 23 lens; 24 fitting groove; 26 simulator; 27 reflector; 31 Y-connector; 32 input port; 33 input port; 34 output port; 40 drive control device; 41 insertion foot switch; 42 line; 43 insertion speed instruction portion; 44 line; 45, 50 volume switch; 46 pull-out foot switch; 47 line; 48 pull-out speed instruction portion; 49 line; 51 housing; 52 main body; 53 through hole; 54A, 54B input/output port; 55A, 55B restraint portion; 56 detection portion; 57, 58 inner wall; 59 recess portion; 60 measurement device; 61 inner wall; 62 measurement device; 63, 64 boundary portion; 65 illumination circuit; 66 conversion circuit; 67 linear body selector; 71 speed control portion; 72 current detector; 73 motor; 74 encoder; 75 speed detection portion; 76 driving force conversion portion; 77 driving force display portion; 80 line sensor; 81 light source; 82 coil portion; 83 delivery wire portion; 84 sheath groove; 85 sheath; 90 sensor output control device; 91 line; 92 speaker; 93 display; 94 warning indicator; 100 medical appliance; 101 coil; 102 microcatheter; 103 guiding catheter; 104 delivery wire; 105, 106 holding portion; 111, 121 Y-connector; 112, 122 input port; 131 human body; 132 blood vessel; 133 aneurysm; 133 cerebral aneurysm; 134 high-coil-density region; 135 low-coil-density region; 201 video camera; 202 microphone; 203, 204 modulator; 205 mixer; 206 video recording device; 207 video monitor; 208 speaker; 209 separator; 210, 211 demodulator; 212 acting force display portion; 213 drive speed display portion; 301 linear body operation recording system; 302 training device; and 501 to 505 insertion device.

### ITEMS

1. An insertion device for inserting a medical linear body (104) in a vessel in a body, comprising:
   a drive device (1) for moving said medical linear body (104) in a direction of a longitudinal axis;
   a measurement device (60) for measuring compressive force and pulling force in the direction of the longitudinal axis applied to said medical linear body (104); and a notification device (92, 93) for notifying an operator of compressive force and pulling force measured with said measurement device (60).
2. The insertion device according to item 1, further comprising a control device (40) for controlling, when compressive force measured with said measurement device (60) exceeds a first threshold value or when pulling force measured with said measurement device (60) exceeds a second threshold value, said drive device (1) so as to reduce a moving speed of said medical linear body (104) in accordance with an amount of excess of said compressive force and said pulling force over respective said first and second threshold values or stop movement of said medical linear body (104).
3. The insertion device according to item 1, further comprising a Y-connector (31) for inserting said medical linear body (104) in a catheter, wherein
   said drive device (1) is arranged on a side of a port for insertion of said medical linear body (104) in said Y-connector (31) and is attachable to and removable from said Y-connector (31).
4. The insertion device according to item 1, wherein
   said medical linear body (104) is a delivery wire (104) provided with an embolus coil (101) at a tip end.
5. The insertion device according to item 1, further comprising a foot switch (41, 46) for starting and stopping movement of said medical linear body (104) by said drive device (1) in response to an operation by said operator.
6. The insertion device according to item 1, wherein
   said notification device (92, 93) notifies the operator of compressive force and pulling force applied to said medical linear body (104) through light or sound and changes luminance of light, a wavelength of light, a frequency of light, the number of light beams intermittently emitted per a prescribed time period, sound volume, a wavelength of sound, a frequency of sound, or the number of sounds intermittently emitted per a prescribed time period in accordance with magnitude of compressive force and pulling force measured with said measurement device (60).
7. The insertion device according to item 1, wherein
   said measurement device (60) includes a main body (52) in which a through hole (53) for inserting said medical linear body (104) is formed,
   said through hole (53) is formed to allow bending of said medical linear body (104) in an arc shape in said through hole (53) and variation in degree of bending of said medical linear body (104) in accordance with said compressive force and said pulling force,
   said measurement device (60) further includes
   a sensor (80) for detecting a degree of bending of said medical linear body (104), and
   a conversion circuit (66) for converting said degree of bending detected by said sensor (80) into a signal indicating compressive force and pulling force applied to said medical linear body (104), and
   said conversion circuit (66) converts detected said degree of bending into a signal indicating compressive force applied to said medical linear body (104) when the degree of bending of said medical linear body (104) increases as compared with the degree of bending of said medical linear body (104) while compressive force and pulling force are not applied to said medical linear body (104), and converts detected said degree of bending into a signal indicating pulling force applied to said medical linear body (104) when the degree of bending of said medical linear body (104) decreases as compared with the degree of bending of said medical linear body (104) while compressive force and pulling force are not applied to said medical linear body (104).
8. The insertion device according to item 7, further comprising a Y-connector (31) for inserting said medical linear body (104) in a catheter, wherein
   said measurement device (60) is integrated with said Y-connector (31).
9. The insertion device according to item 7, wherein
   said sensor (80) is an optical line sensor (80) including a light receiver (80) receiving light emitted by a light source (81), for detecting the degree of bending of said medical linear body (104) by detecting a position at which a quantity of light received by said light receiver (81) decreases as said medical linear body (104) cuts off the light emitted by said light source (81).
10. The insertion device according to item 9, further comprising a Y-connector (31) for inserting said medical linear body (104) in a catheter, wherein
   said Y-connector (31) is attachable to and removable from said line sensor (80).
11. The insertion device according to item 9, wherein
   said line sensor (80) and said drive device (1) are integrated with each other with a base (22) interposed therebetween.
12. The insertion device according to item 1, wherein
   said drive device (1) includes
   a rotational force generator (3),
   a drive roller (5) rotatably driven by rotational force generated by said rotational force generator,
   a driven roller (6) rotated as said drive roller (5) rotates,
   a case (2) containing said rotational force generator (3), said drive roller (5), and said driven roller (6), and
   an elastic body (8) provided between said case (2) and said driven roller (6),
   said medical linear body (104) is sandwiched between a rotation surface of said drive roller (5) and a rotation surface of said driven roller (6),
   said driven roller (6) is supported by said case (2) with said elastic body (8) being interposed, and pressed against said drive roller (5) by elastic force from said elastic body (8), and
   pressing of said driven roller (6) against said drive roller (5) can manually be removed.
13. The insertion device according to item 1, wherein
   said drive device (1) includes
   a rotational force generator (73) for generating rotational force for moving said medical linear body (104) in the direction of the longitudinal axis based on a supplied drive current, and
   a current detector (72) for detecting said drive current, and
   said measurement device (76) measures compressive force and pulling force applied to said medical linear body (104) based on said drive current detected by said current detector (72).
14. The insertion device according to item 1, further comprising a linear body speed instruction portion (43, 48) for controlling a speed of said medical linear body (104) moved by said drive device (1) in response to an operation by said operator.
15. A training device comprising the insertion device according to item 1.
16. A recording system, comprising:
   an insertion device for inserting a medical linear body (104) in a vessel in a body;and
   a recording device (206),
   said insertion device including
      a drive device (1) for moving said medical linear body (104) in a direction of a longitudinal axis,
      a measurement device (60) for measuring compressive force and pulling force in the direction of the longitudinal axis applied to said medical linear body (104), and
      a notification device (92, 93) for notifying an operator of compressive force and pulling force measured with said measurement device (60), and
   said recording device (206) recording an image obtained by photographing said medical linear body (104) as well as compressive force and pulling force measured with said measurement device (60), temporally in correspondence with each other.
17. The recording system according to item 16, wherein
   said recording device (206) records said image, said compressive force and pulling force, and a speed of said medical linear body (104) moved by said drive device (1), temporally in correspondence with one another.
18. An insertion device for inserting a medical linear body (104) in a vessel in a body, comprising:
   a drive device (1) for moving said medical linear body (104) in a direction of a longitudinal axis;
   a measurement device (60) for measuring compressive force and pulling force in the direction of the longitudinal axis applied to said medical linear body (104);
   a notification device (92, 93) for notifying an operator of compressive force and pulling force measured with said measurement device (60); and
   a foot switch (41) for starting and stopping movement of said medical linear body (104) by said drive device (1) in response to an operation by said operator.
19. The insertion device according to item 18, further comprising a control device (40) for controlling, when compressive force measured with said measurement device (60) exceeds a first threshold value or when pulling force measured with said measurement device (60) exceeds a second threshold value, said drive device (1) so as to reduce a moving speed of said medical linear body (104) in accordance with an amount of excess of said compressive force and said pulling force over respective said first and second threshold values or stop movement of said medical linear body (104).
20. The insertion device according to item 18, further comprising a Y-connector (31) for inserting said medical linear body (104) in a catheter, wherein
   said drive device (1) is arranged on a side of a port for insertion of said medical linear body (104) in said Y-connector (31) and is attachable to and removable from said Y-connector (31).
21. The insertion device according to item 18, wherein
   said medical linear body (104) is a delivery wire (104) provided with an embolus coil (101) at a tip end.
22. The insertion device according to item 18, wherein
   said drive device (1) selectively moves said medical linear body (104) in any direction of a first direction of insertion in the vessel in said body and a second direction of pulling out of the vessel in said body,
   said foot switch (41) starts and stops movement in said first direction of said medical linear body (104) by said drive device (1) in response to the operation by said operator, and
   said insertion device further comprises another foot switch (46) for starting and stopping movement in said second direction of said medical linear body (104) by said drive device (1) in response to an operation by said operator.
23. The insertion device according to item 18, wherein
   said notification device (92, 93) notifies the operator of compressive force and pulling force applied to said medical linear body (104) through light or sound and changes luminance of light, a wavelength of light, a frequency of light, the number of light beams intermittently emitted per a prescribed time period, sound volume, a wavelength of sound, a frequency of sound, or the number of sounds intermittently emitted per a prescribed time period in accordance with magnitude of compressive force and pulling force measured with said measurement device (60).
24. The insertion device according to item 18, wherein
   said measurement device (60) includes a main body (52) in which a through hole (53) for inserting said medical linear body (104) is formed,
   said through hole (53) is formed to allow bending of said medical linear body (104) in an arc shape in said through hole (53) and variation in degree of bending of said medical linear body (104) in accordance with said compressive force and said pulling force,
   said measurement device (60) further includes
   a sensor (80) for detecting a degree of bending of said medical linear body (104), and
   a conversion circuit (66) for converting said degree of bending detected by said sensor (80) into a signal indicating compressive force and pulling force applied to said medical linear body (104), and
   said conversion circuit (66) converts detected said degree of bending into a signal indicating compressive force applied to said medical linear body (104) when the degree of bending of said medical linear body (104) increases as compared with the degree of bending of said medical linear body (104) while compressive force and pulling force are not applied to said medical linear body (104), and converts detected said degree of bending into a signal indicating pulling force applied to said medical linear body (104) when the degree of bending of said medical linear body (104) decreases as compared with the degree of bending of said medical linear body (104) while compressive force and pulling force are not applied to said medical linear body (104).
25. The insertion device according to item 24, further comprising a Y-connector (31) for inserting said medical linear body (104) in a catheter, wherein
   said measurement device (60) is integrated with said Y-connector (31).
26. The insertion device according to item 24, wherein
   said sensor (80) is an optical line sensor (80) including a light receiver (80) receiving light emitted by a light source (81), for detecting the degree of bending of said medical linear body (104) by detecting a position at which a quantity of light received by said light receiver (81) decreases as said medical linear body (104) cuts off the light emitted by said light source (81).
27. The insertion device according to item 26, further comprising a Y-connector (31) for inserting said medical linear body (104) in a catheter, wherein
   said Y-connector (31) is attachable to and removable from said line sensor (80).
28. The insertion device according to item 26, wherein
   said line sensor (80) and said drive device (1) are integrated with each other with a base (22) interposed therebetween.
29. The insertion device according to item 18, wherein
   said drive device (1) includes
   a rotational force generator (3),
   a drive roller (5) rotatably driven by rotational force generated by said rotational force generator,
   a driven roller (6) rotated as said drive roller (5) rotates,
   a case (2) containing said rotational force generator (3), said drive roller (5), and said driven roller (6), and
   an elastic body (8) provided between said case (2) and said driven roller (6),
   said medical linear body (104) is sandwiched between a rotation surface of said drive roller (5) and a rotation surface of said driven roller (6),
   said driven roller (6) is supported by said case (2) with said elastic body (8) being interposed, and pressed against said drive roller (5) by elastic force from said elastic body (8), and
   pressing of said driven roller (6) against said drive roller (5) can manually be removed.
30. The insertion device according to item 18, wherein
   said drive device (1) includes
   a rotational force generator (73) for generating rotational force for moving said medical linear body (104) in the direction of the longitudinal axis based on a supplied drive current, and
   a current detector (72) for detecting said drive current, and
   said measurement device (76) measures compressive force and pulling force applied to said medical linear body (104) based on said drive current detected by said current detector (72).
31. The insertion device according to item 18, further comprising a linear body speed instruction portion (43, 48) for controlling a speed of said medical linear body (104) moved by said drive device (1) in response to an operation by said operator.
32. A training device comprising the insertion device according to item 18.
33. A recording system, comprising:
   an insertion device for inserting a medical linear body (104) in a vessel in a body;and
   a recording device (206),
   said insertion device being the insertion device according to item 18, and
   said recording device (206) recording an image obtained by photographing said medical linear body (104) as well as compressive force and pulling force measured with said measurement device (60), temporally in correspondence with each other.
34. The recording system according to item 33, wherein
   said recording device (206) records said image, said compressive force and pulling force, and a speed of said medical linear body (104) moved by said drive device (1), temporally in correspondence with one another.

## Claims

1. An insertion device for inserting a medical linear body (104) in a vessel in a body, comprising:
a drive device (1) for moving said medical linear body (104) in a direction of a longitudinal axis;
a measurement device (60) for measuring compressive force and pulling force in the direction of the longitudinal axis applied to said medical linear body (104);
a notification device (92, 93) for notifying an operator of compressive force and pulling force measured with said measurement device (60); and a control device (40) adapted for controlling, when compressive force measured with said measurement device (60) exceeds a first threshold value or when pulling force measured with said measurement device (60) exceeds a second threshold value, said drive device (1) adapted for reducing a moving speed of said medical linear body (104) in accordance with an amount of excess of said compressive force and said pulling force over respective said first and second threshold values or stop movement of said medical linear body (104).

2. The insertion device according to claim 1, further comprising a foot switch (41) for starting and stopping movement of said medical linear body (104) by said drive device (1) in response to an operation by said operator.

3. The insertion device according to claim 1, further comprising a Y-connector (31) for inserting said medical linear body (104) in a catheter, wherein said drive device (1) is arranged on a side of a port for insertion of said medical linear body (104) in said Y-connector (31) and is attachable to and removable from said Y-connector (31).

4. The insertion device according to claim 1, wherein
said medical linear body (104) is a delivery wire (104) provided with an embolus coil (101) at a tip end.

5. The insertion device according to claim 2, wherein
said drive device (1) adapted for selectively moving said medical linear body (104) in any direction of a first direction of insertion in the vessel in said body and a second direction of pulling out of the vessel in said body,
said foot switch (41) adapted for starting and stopping movement in said first direction of said medical linear body (104) by said drive device (1) in response to the operation by said operator, and
said insertion device further comprises another foot switch (46) for starting and stopping movement in said second direction of said medical linear body (104) by said drive device (1) in response to an operation by said operator.

6. The insertion device according to claim 1, wherein
said notification device (92, 93) adapted for notifying the operator of compressive force and pulling force applied to said medical linear body (104) through light or sound and to changing luminance of light, a wavelength of light, a frequency of light, the number of light beams intermittently emitted per a prescribed time period, sound volume, a wavelength of sound, a frequency of sound, or the number of sounds intermittently emitted per a prescribed time period in accordance with magnitude of compressive force and pulling force measured with said measurement device (60).

7. The insertion device according to claim 1, wherein
said measurement device (60) includes a main body (52) in which a through hole (53) for inserting said medical linear body (104) is formed,
said through hole (53) is formed to allow bending of said medical linear body (104) in an arc shape in said through hole (53) and variation in degree of bending of said medical linear body (104) in accordance with said compressive force and said pulling force,
said measurement device (60) further includes
a sensor (80) for detecting a degree of bending of said medical linear body (104), and
a conversion circuit (66) for converting said degree of bending detected by said sensor (80) into a signal indicating compressive force and pulling force applied to said medical linear body (104), and
said conversion circuit (66) converts detected said degree of bending into a signal indicating compressive force applied to said medical linear body (104) when the degree of bending of said medical linear body (104) increases as compared with the degree of bending of said medical linear body (104) while compressive force and pulling force are not applied to said medical linear body (104), and converts detected said degree of bending into a signal indicating pulling force applied to said medical linear body (104) when the degree of bending of said medical linear body (104) decreases as compared with the degree of bending of said medical linear body (104) while compressive force and pulling force are not applied to said medical linear body (104).

8. The insertion device according to claim 7, further comprising a Y-connector (31) for inserting said medical linear body (104) in a catheter, wherein
said measurement device (60) is integrated with said Y-connector (31).

9. The insertion device according to claim 7, wherein
said sensor (80) is an optical line sensor (80) including a light receiver (80) receiving light emitted by a light source (81), for detecting the degree of bending of said medical linear body (104) by detecting a position at which a quantity of light received by said light receiver (81) decreases as said medical linear body (104) cuts off the light emitted by said light source (81).

10. The insertion device according to claim 9, further comprising a Y-connector (31) for inserting said medical linear body (104) in a catheter, wherein
said Y-connector (31) is attachable to and removable from said line sensor (80).

11. The insertion device according to claim 9, wherein
said line sensor (80) and said drive device (1) are integrated with each other with a base (22) interposed therebetween.

12. The insertion device according to claim 1, wherein
said drive device (1) includes
a rotational force generator (3),
a drive roller (5) rotatably driven by rotational force generated by said rotational force generator,
a driven roller (6) rotated as said drive roller (5) rotates,
a case (2) containing said rotational force generator (3), said drive roller (5), and said driven roller (6), and
an elastic body (8) provided between said case (2) and said driven roller (6),
said medical linear body (104) is sandwiched between a rotation surface of said drive roller (5) and a rotation surface of said driven roller (6),
said driven roller (6) is supported by said case (2) with said elastic body (8) being interposed, and pressed against said drive roller (5) by elastic force from said elastic body (8), and
pressing of said driven roller (6) against said drive roller (5) can manually be removed.

13. The insertion device according to claim 1, wherein
said drive device (1) includes
a rotational force generator (73) for generating rotational force for moving said medical linear body (104) in the direction of the longitudinal axis based on a supplied drive current, and
a current detector (72) for detecting said drive current, and
said measurement device (76) measures compressive force and pulling force applied to said medical linear body (104) based on said drive current detected by said current detector (72).

14. The insertion device according to claim 1, further comprising a linear body speed instruction portion (43, 48) adapted for controlling a speed of said medical linear body (104) moved by said drive device (1) in response to an operation by said operator.

15. A training device comprising the insertion device according to claim 1.

16. A recording system, comprising:
an insertion device for inserting a medical linear body (104) in a vessel in a body; and
a recording device (206),
said insertion device being the insertion device according to claim 1, and
said recording device (206) being adapted for recording an image obtained by photographing said medical linear body (104) as well as compressive force and pulling force measured with said measurement device (60), temporally in correspondence with each other.

17. The recording system according to claim 16, wherein
said recording device (206) being adapted for recording said image, said compressive force and pulling force, and a speed of said medical linear body (104) moved by said drive device (1), temporally in correspondence with one another.
